(19) Europäisches Patentamt European Patent Office Office européen des brevets

(11) **EP 4 071 657 B1**

(12) **EUROPEAN PATENT SPECIFICATION**

(45) Date of publication and mention of the grant of the patent:
**11.12.2024 Bulletin 2024/50**

(21) Application number: **22161146.0**

(22) Date of filing: **09.03.2022**

(51) International Patent Classification (IPC):
*G06F 30/27* (2020.01)    *G06N 3/09* (2023.01)
*G06N 3/048* (2023.01)    *G06N 3/0475* (2023.01)
*G06N 3/047* (2023.01)    *G06N 3/0464* (2023.01)
*G06N 3/0455* (2023.01)    *G16C 60/00* (2019.01)

(52) Cooperative Patent Classification (CPC):
**G06F 30/27; G06N 3/0455; G06N 3/0464;**
**G06N 3/047; G06N 3/0475; G06N 3/048;**
**G06N 3/09; G16C 60/00;** G16C 20/70; G16C 20/80

(54) **SYSTEMS AND METHODS FOR DESIGN OF APPLICATION SPECIFIC FUNCTIONAL MATERIALS**

SYSTEME UND VERFAHREN ZUM ENTWURF VON ANWENDUNGSSPEZIFISCHEN FUNKTIONELLEN MATERIALIEN

SYSTÈMES ET PROCÉDÉS DE CONCEPTION DE MATÉRIAUX FONCTIONNELS SPÉCIFIQUES À UNE APPLICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.04.2021 IN 202121016622**

(43) Date of publication of application:
**12.10.2022 Bulletin 2022/41**

(73) Proprietor: **Tata Consultancy Services Limited Maharashtra (IN)**

(72) Inventors:
• **AGARWAL, ABHISHEK**
  **411013 Pune, Maharashtra (IN)**
• **GOVERAPET SRINIVASAN, SRIRAM**
  **411013 Pune, Maharashtra (IN)**
• **RAI, BEENA**
  **411013 Pune, Maharashtra (IN)**

(74) Representative: **Goddar, Heinz J.**
  **Boehmert & Boehmert**
  **Anwaltspartnerschaft mbB**
  **Pettenkoferstrasse 22**
  **80336 München (DE)**

(56) References cited:
• **TENG LONG ET AL: "CCDCGAN: Inverse design of crystal structures", ARXIV.ORG, CORNELL UNIVERSITY LIBRARY, 201 OLIN LIBRARY CORNELL UNIVERSITY ITHACA, NY 14853, 22 July 2020 (2020-07-22), XP081725247**
• **SUNGWON KIM ET AL: "Generative Adversarial Networks for Crystal Structure Prediction", ARXIV.ORG, CORNELL UNIVERSITY LIBRARY, 201 OLIN LIBRARY CORNELL UNIVERSITY ITHACA, NY 14853, 3 April 2020 (2020-04-03), XP081687580**
• **MATTHEW SPELLINGS ET AL: "Machine learning for crystal identification and discovery", AICHE JOURNAL, JOHN WILEY & SONS, INC, US, vol. 64, no. 6, 30 March 2018 (2018-03-30), pages 2198 - 2206, XP071007143, ISSN: 0001-1541, DOI: 10.1002/AIC.16157**
• **YONG ZHAO ET AL: "High-throughput discovery of novel cubic crystal materials using deep generative neural networks", ARXIV.ORG, CORNELL UNIVERSITY LIBRARY, 201 OLIN LIBRARY CORNELL UNIVERSITY ITHACA, NY 14853, 3 February 2021 (2021-02-03), XP081874265**
• **ZEKUN REN ET AL: "Inverse design of crystals using generalized invertible crystallographic representation", ARXIV.ORG, CORNELL UNIVERSITY LIBRARY, 201 OLIN LIBRARY CORNELL UNIVERSITY ITHACA, NY 14853, 22 June 2020 (2020-06-22), XP081688786**

EP 4 071 657 B1

- ASMA NOUIRA ET AL: "CrystalGAN: Learning to Discover Crystallographic Structures with Generative Adversarial Networks", ARXIV.ORG, CORNELL UNIVERSITY LIBRARY, 201 OLIN LIBRARY CORNELL UNIVERSITY ITHACA, NY 14853, 26 October 2018 (2018-10-26), XP081070514
- JUHWAN NOH: "Inverse Design of Solid-State Materials via a Continuous Representation", MATTER, vol. 1, no. 5, 6 November 2019 (2019-11-06), US, pages 1370 - 1384, XP093157341, ISSN: 2590-2385, Retrieved from the Internet <URL:https://dx.doi.org/10.1016/j.matt.2019.08.017> DOI: 10.1016/j.matt.2019.08.017
- JORDAN HOFFMANN ET AL: "Data-Driven Approach to Encoding and Decoding 3-D Crystal Structures", ARXIV.ORG, CORNELL UNIVERSITY LIBRARY, 201 OLIN LIBRARY CORNELL UNIVERSITY ITHACA, NY 14853, 3 September 2019 (2019-09-03), XP081472288
- COURT CALLUM J ET AL: "3-D Inorganic Crystal Structure Generation and Property Prediction via Representation Learning", JOURNAL OF CHEMICAL INFORMATION AND MODELING, vol. 60, no. 10, 31 August 2020 (2020-08-31), US, pages 4518 - 4535, XP093174605, ISSN: 1549-9596, Retrieved from the Internet <URL:https://pubs.acs.org/doi/pdf/10.1021/acs.jcim.0c00464> DOI: 10.1021/acs.jcim.0c00464

**Description**

CROSS-REFERENCE TO RELATED APPLICATIONS AND PRIORITY

The present application claims priority to Indian application no. 202121016622, filed on April 08, 2021.

TECHNICAL FIELD

The disclosure herein generally relates to new material designing for various applications, and, more particularly, to system and method for designing of new materials based on the application properties of such materials.

BACKGROUND

Discovery and design of novel materials with enhanced efficiency is essential for a green and sustainable future. Much of materials discovery, design and deployment in the past has been via experimentation, rendering the material development process extremely slow. With advent of AI and the availability of large and cheap computational power, material property data computed via first principles calculations are being leveraged to predict the property of a given material. However, these methods are forward models that are incapable of 'custom material discovery/design', i.e., development of materials given a target property / end use, also known as 'inverse design'.

Document ("Inverse Design of Solid-State Materials via a Continuous Representation", Matter, vol. 1, no. 5, 6 November 2019, pages 1370-1384, US ISSN: 2590-2385, DOI: 10.1016/j.matt.2019.08.017) discloses a generative model using invertible image-based representation yields accurate reconstruction performance and can successfully rediscover experimentally known vanadium oxides. The model predicts several completely new compositions and polymorphs of vanadium oxides that are metastable and may be synthesizable.

Document ("CCDCGAN: Inverse design of crystal structures" ARXIV.ORG, CORNELL UNIVERSITY LIBRARY) describes autonomous materials discovery with desired properties is one of the ultimate goals for modern materials science. Applying the deep learning techniques, a generative model is developed which can predict distinct stable crystal structures by optimizing the formation energy in the latent space. Further, the optimization of physical properties can be integrated into the generative model as on-top screening or backwards propagator, both with their own advantages. Applying the generative models on the binary Bi-Se system reveals that distinct crystal structures can be obtained covering the whole composition range, and the phases on the convex hull can be reproduced after the generated structures are fully relaxed to the equilibrium. The method can be extended to multicomponent systems for multi-objective optimization, which paves the way to achieve the inverse design of materials with optimal properties.

Document ("Generative Adversarial Networks for Crystal Structure Prediction", ARXIV.ORG, CORNELL UNIVERSITY LIBRARY) describes strategies to accelerate the materials discovery, and crystal structure prediction is one of the most fundamental tasks along that direction. In addressing this challenge, generative models can offer new opportunities since they allow for the continuous navigation of chemical space via latent spaces. A crystal representation that is inversion-free based on unit cell and fractional atomic coordinates, and build generative adversarial network for crystal structures. The proposed model is applied to generate the MgMn-O ternary materials with the theoretical evaluation of their photoanode properties for high throughput virtual screening (HTVS). The proposed generative HTVS framework predicts 23 new crystal structures with reasonable calculated stability and bandgap. These findings suggest that the generative model can be an effective way to explore hidden portions of the chemical space, an area that is usually unreachable when conventional substitution-based discovery is employed.

Document (Machine learning for crystal identification and discovery", AICHE JOURNAL, JOHN WILEY & SONS, INC,) describes how machine learning can be applied to discover interesting areas of parameter space in colloidal self-assembly. We create numerical fingerprints-inspired by bond orientational order diagrams-of structures found in self-assembly studies and use these descriptors to both find interesting regions in a phase diagram and identify characteristic local environments in simulations in an automated manner for simple and complex crystal structures. Utilizing these methods allows analysis to keep up with the data generation ability of modern high-throughput computing environments.

Document (High-throughput discovery of novel cubic crystal materials using deep generative neural networks", ARXIV.ORG, CORNELL UNIVERSITY LIBRARY) describes a generative adversarial network (GAN) based deep neural network model for large scale generation of novel cubic crystal structures. When trained on 375,749 ternary crystal materials from the OQMD database, the model is able to not only rediscover most of the currently known cubic materials but also generate hypothetical materials of new structure prototypes. A total of 506 such new materials (all of them are either ternary or quarternary) have been verified by DFT based phonon dispersion stability check, several of which have been found to potentially have exceptional functional properties. Considering the importance of cubic materials in wide applications such as solar cells and lithium batteries, our GAN model provides a promising approach to significantly expand the current repository of materials, enabling the discovery of new functional materials via screening.

Document (Inverse design of crystals using generalized invertible crystallographic representation", ARXIV.ORG, CORNELL UNIVERSITY LIBRARY) describes framework capable of general inverse design (not limited to a given set of elements or crystal structures), featuring a generalized invertible representation that encodes crystals in both real and reciprocal space, and a property-structured latent space from a variational autoencoder (VAE). In three design cases, the framework generates 142 new crystals with user-defined formation energies, bandgap, thermoelectric (TE) power factor, and combinations thereof. These generated crystals, absent in the training database, are validated by first-principles calculations. The success rates (number of first-principles-validated target-satisfying crystals/number of designed crystals) ranges between 7.1% and 38.9%. These results represent a significant step toward property-driven general inverse design using generative models, although practical challenges remain when coupled with experimental synthesis.

Document ("CrystalGAN: Learning to Discover Crystallographic Structures with Generative Adversarial Networks", ARXIV.ORG, CORNELL UNIVERSITY LIBRARY,) describes a novel GAN called CrystalGAN which generates new chemically stable crystallographic structures with increased domain complexity. The system introduce an original architecture, proving the corresponding loss functions, and we show that the CrystalGAN generates very reasonable data. The system illustrate the efficiency of the proposed method on a real original problem of novel hydrides discovery that can be further used in development of hydrogen storage materials.

SUMMARY

Embodiments of the present disclosure present technological improvements as solutions to one or more of the above-mentioned technical problems recognized by the inventors in conventional systems. For example, in one embodiment, a processor implemented method is provided. The method includes obtaining crystal structure of each of a plurality of materials obtained in a training data set, via one or more hardware processors. Further, the method includes converting the crystal structure of each material of the plurality of materials into a three-dimensional (3D) cell image and a 3D basis image using a plurality of gaussian functions, via the one or more hardware processors. Also, the method includes creating, for each 3D basis image of the each material, a 3D elements matrix representing location of one or more elements in the 3D basis image of the material, via the one or more hardware processors. Moreover, the method includes training, via the one or more hardware processors, a basis autoencoder using the 3D basis image of the each material and obtaining a set of reconstructed basis images. Also, the method includes training, via the one or more hardware processors, a segmentation network using the set of reconstructed basis images to identify location and types of a set of elements at the locations as atomic clusters, the segmentation network trained by using a species matrix for each material as a ground truth wherein the species matrix is determined using the 3D elements matrix for the material. Also, the method includes training a cell autoencoder using the 3D cell image of the each material and obtaining a set of reconstructed cell images, via the one or more hardware processors. The method then includes training, using the set of reconstructed cell images and the set of reconstructed basis images, a generative model to obtain a continuous latent space, via the one or more hardware processors. The method then include sampling, via the one or more hardware processors, the continuous latent space of the generative model to obtain a set of cell encoding and a set of basis encoding for one or more new materials associated with one or more conditions of the application, the sampling performed using one of a random sampling or interpolating between latent vectors of one or more materials from amongst the plurality of known materials using one of a spherical and linear interpolation (SLERP) techniques. Also, the method includes passing, via the one or more hardware processors, the set of cell encoding through the cell autoencoder to obtain a set of sampled cell images and the set of basis encodings through the basis autoencoder to obtain a set of sampled basis images. Then the method includes inverting the set of sampled cell images to obtain a set of lattice vectors for the one or more new materials, via the one or more hardware processors. Finally, the method includes passing, via the one or more hardware processors, the set of sampled basis images through the segmentation network to obtain a set of atomic clusters, wherein the set of atomic clusters are indicative of atomic positions and element types at the atomic positions, and wherein atom coordinates from the set of atomic clusters combined with the set of lattice vectors constitutes the crystal structure of the one or more new materials.

In another aspect, a system is provided. The system includes a memory storing instructions; one or more communication interfaces; and one or more hardware processors coupled to the memory via the one or more communication interfaces, wherein the one or more hardware processors are configured by the instructions to obtain crystal structure of each of a plurality of materials obtained in a training data set. The one or more hardware processors are further configured by the instructions to convert the crystal structure of each material of the plurality of materials into a three-dimensional (3D) cell image and a 3D basis image using a plurality of gaussian functions. The one or more hardware processors are further configured by the instructions to create, for each 3D basis image of the each material, a 3D elements matrix representing location of one or more elements in the 3D basis image of the material. The one or more hardware processors are further configured by the instructions to train a basis autoencoder using the 3D basis image of the each material and obtaining a set of reconstructed basis images. The one or more hardware processors are further configured by the instructions to train a segmentation network using the set of reconstructed basis images to identify location and types of a set of elements at the locations as atomic clusters, the segmentation network trained by using a species matrix for each material as a ground truth wherein the species matrix is determined using the 3D elements matrix for the material. The one or more hardware processors are further configured by the instructions to train a cell autoencoder using the 3D cell image of the each material and obtaining a set of reconstructed cell images. The one or more hardware processors are further configured by the instructions to train, using the set of reconstructed cell images and the set of reconstructed basis images, a generative model to obtain a continuous latent space. The one or more hardware processors are further configured by the instructions to sample the continuous latent space of the generative model to obtain a set of cell encoding and a set of basis encoding for one or more new materials associated with one or more conditions of the application, the sampling performed using one of a random sampling or interpolating between latent vectors of one or more materials from amongst the plurality of known materials using one of a spherical and linear interpolation (SLERP) techniques. The one or more hardware processors are further configured by the instructions to pass the set of cell encoding through the cell autoencoder to obtain a set of sampled cell images and the set of basis encodings through the basis autoencoder to obtain a set of sampled basis images. The one or more hardware processors are further configured by the instructions to invert the set of sampled cell images to obtain a set of lattice vectors for the one or more new materials. The one or more hardware processors are further configured by the instructions to pass the set of sampled basis images through the segmentation network to obtain a set of atomic clusters, wherein the set of atomic clusters are indicative of atomic positions and element types at the atomic positions, and wherein atom coordinates from the set of atomic clusters combined with the set of lattice vectors constitutes the crystal structure of the one or more new materials.

In yet another aspect, a non-transitory computer readable medium for a method. The method includes obtaining crystal structure of each of a plurality of materials obtained in a training data set, via one or more hardware processors. Further, the method includes converting the crystal structure of each material of the plurality of materials into a three-dimensional (3D) cell image and a 3D basis image using a plurality of gaussian functions, via the one or more hardware processors. Also, the method includes creating, for each 3D basis image of the each material, a 3D elements matrix representing location of one or more elements in the 3D basis image of the material, via the one or more hardware processors. Moreover, the method includes training, via the one or more hardware processors, a basis autoencoder using the 3D basis image of the each material and obtaining a set of reconstructed basis images. Also, the method includes training, via the one or more hardware processors, a segmentation network using the set of reconstructed basis images to identify location and types of a set of elements at the locations as atomic clusters, the segmentation network trained by using a species matrix for each material as a ground truth wherein the species matrix is determined using the 3D elements matrix for the material. Also, the method includes training a cell autoencoder using the 3D cell image of the each material and obtaining a set of reconstructed cell images, via the one or more hardware processors. The method then includes training, using the set of reconstructed cell images and the set of reconstructed basis images, a generative model to obtain a continuous latent space, via the one or more hardware processors. The method then include sampling, via the one or more hardware processors, the continuous latent space of the generative model to obtain a set of cell encoding and a set of basis encoding for one or more new materials associated with one or more conditions of the application, the sampling performed using one of a random sampling or interpolating between latent vectors of one or more materials from amongst the plurality of known materials using one of a spherical and linear interpolation (SLERP) techniques. Also, the method includes passing, via the one or more hardware processors, the set of cell encoding through the cell autoencoder to obtain a set of sampled cell images and the set of basis encodings through the basis autoencoder to obtain a set of sampled basis images. Then the method includes inverting the set of sampled cell images to obtain a set of lattice vectors for the one or more new materials, via the one or more hardware processors. Finally, the method includes passing, via the one or more hardware processors, the set of sampled basis images through the segmentation network to obtain a set of atomic clusters, wherein the set of atomic clusters are indicative of atomic positions and element types at the atomic positions, and wherein atom coordinates from the set of atomic clusters combined with the set of lattice vectors constitutes the crystal structure of the one or more new materials.

It is to be understood that both the foregoing general description and the following detailed description are exemplary and explanatory only and are not restrictive of the invention, as claimed. The present invention is defined in the independent claims. Preferred embodiments are defined in the dependent claims.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an example network implementation of a system for system and method for application based designing of new functional materials, in accordance with an example embodiment.

FIG. 2 illustrates an architecture diagram of the system of FIG. 1 for application based designing of new functional materials, in accordance with an example embodiment.

FIG. 3 is a flow diagram of a method for application based designing of new functional materials, in accordance with an example embodiment.

FIGS. 4A-4B is a flow diagram of application based designing of new functional materials, in accordance with an example embodiment.

FIG. 5 is a block diagram of an exemplary computer system for implementing embodiments consistent with the present disclosure.

FIG. 6 is a representation of cell image and basis image, and elements matrix constructed from a material's crystal structure for application based designing of new functional materials, in accordance with an example scenario.

FIG. 7 is a representation of an input cell image and its comparison with the corresponding output cell image from a cell autoencoder for application based designing of new functional materials, in accordance with an example scenario.

FIG. 8 is a representation of an input and output basis image as well as the elements matrix from the basis autoencoder and segmentation network respectively, in accordance with an example scenario.

FIG. 9 is a graphical representation of performance of a segmentation network for application based designing of new functional materials in accordance with an example scenario.

FIG. 10 illustrates predicted crystal structures of the new functional materials predicted using the system of FIG. 1, in accordance with an example scenario.

DETAILED DESCRIPTION OF EMBODIMENTS

In past, much of materials discovery, design and deployment has been via experimentation, rendering the material development process extremely slow. With advent of AI and the availability of large and cheap computational power, material property data computed via first principles calculations are being leveraged to predict the property of a given material. However, these methods are forward models that are incapable of 'custom material discovery/design', i.e., development of materials given a target property / end use, also known as 'inverse design'.

A known system first uses an images-based representation for crystal structures. However, while representing the basis, this conventional technique uses separate images for each element type. Thus, for instance, when a crystal structure has five distinct elements like in the case of high entropy alloy (for example HfYZrVCr), the techniques requires five separate images to represent a basis. Together with cell image, a material would be represented by six images, which leads to huge memory requirement and also many-fold increase in training time. In addition to that, since each element is represented by its own image, it is difficult for this system to learn the chemical environment and neighborhood pattern of each element.

Another known system that uses image representation for crystal structures does not represent cell as separate images but passes cell information in terms of cell parameters (cell lengths and angles). This, however, makes the model incapable of generating crystal structures other than those of cubic symmetry.

Certain other known system uses point cloud-based representation for crystal structures. However, this representation suffers from permutation variance. For example, an $H_2O$ molecule (water) can be represented with different permutations of constituent elements such as HOH, OHH and HHO, all of which physically represent the same $H_2O$ molecule. Since permutation invariance is not inbuilt in a material's representation, the DL model has to learn this invariance which can never be 100% accurate.

Yet another study was focused on generating new structures of Mg-Mn-O system. Such representation clearly lacks generality and is very system specific. Such representation is therefore not feasible for representing a general composition and nearly impossible to consider 118 different elements in periodic table. Although permutation invariance problem was alleviated by data-augmentation, point cloud representation does not include this physical reality by default.

In yet another study, no shuffling of sites in a material was considered to alleviate permutational invariance problem, although a point cloud like representation was used. Also, their study was restricted to at most ternary system. The method cannot be applied directly for quaternary systems since clubbing cell matrix (2,3) with an atomic matrix (K,4) (in case of Quaternary) won't be possible due to incompatibility of the last dimension of the matrices (3 vs 4).

As is seen from above, the conventional method and system suffers from various limitations including, but not limited to, generate new inorganic crystals, limited to few distinct type of atoms, does not involve designing materials for a specific application, i.e., properties are not linked with material generation, and limited to encoding of atom positions and has no realization of crystal structure. As is understood, the crystal structure defines the properties for a material and hence forms the basis for a functional material design. Some of the conventional methods are limited to cubic symmetry of crystal and number of atoms in unit cell, thus are not generic.

The disclosed embodiments provide method and system that overcomes the aforementioned limitations of the known methods and systems for inverse design of functional material. The disclosed methods and systems are not limited to materials of a certain kind (say in terms of the number of atoms or elements or crystal types). Instead, the disclosed methods and systems allow inverse design of materials from a much wider configurational space. In an embodiment, the disclosed system uses deep learning techniques to achieve the objective. The use of deep learning techniques accelerates discovery and design of functional materials, thereby minimizing laborious and costly experiment/first principles-based screening of materials. In addition, the disclosed system and method overcomes the current lack of a generic framework for the inverse design of functional materials.

In an embodiment, the disclosed system represents a crystal structure of the materials in the same way as they are physically construed. Specifically, just as a crystal structure is understood as a 'basis' of atoms in a 'lattice', the disclosed system represents a crystal structure as a combination of 'cell' and 'basis' images, with no restrictions on the shapes of the 'cell' (or lattice) or the number and types of atoms in the basis. In another embodiment, the disclosed method enables property prediction along with materials generation so that new materials can be discovered targeting specific properties as demanded by the end use.

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope being indicated by the following claims.

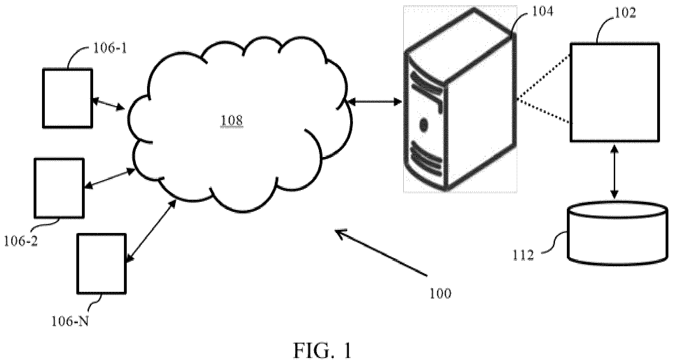

Referring now to the drawings, and more particularly to FIG. 1 through 10, where similar reference characters denote corresponding features consistently throughout the figures, there are shown preferred embodiments and these embodiments are described in the context of the following exemplary system and/or method.

FIG. 1 illustrates an example network implementation 100 of a system 102 for designing of new materials, in accordance with an example embodiment. The disclosed method enables the discovery of new functional materials.

Although the present disclosure is explained considering that the system 102 is implemented on a server, it may be understood that the system 102 may also be implemented in a variety of computing systems 104, such as a laptop computer, a desktop computer, a notebook, a workstation, a cloud-based computing environment and the like. It will be understood that the system 102 may be accessed through one or more devices 106-1, 106-2... 106-N, collectively referred to as devices 106 hereinafter, or applications residing on the devices 106. Examples of the devices 106 may include, but are not limited to, a portable computer, a personal digital assistant, a handheld device, a smartphone, a tablet computer, a workstation and the like. The devices 106 are communicatively coupled to the system 102 through a network 108.

In an embodiment, the network 108 may be a wireless or a wired network, or a combination thereof. In an example, the network 108 can be implemented as a computer network, as one of the different types of networks, such as virtual private network (VPN), intranet, local area network (LAN), wide area network (WAN), the internet, and such. The network 106 may either be a dedicated network or a shared network, which represents an association of the different types of networks that use a variety of protocols, for example, Hypertext Transfer Protocol (HTTP), Transmission Control Protocol/Internet Protocol (TCP/IP), and Wireless Application Protocol (WAP), to communicate with each other. Further, the network 108 may include a variety of network devices, including routers, bridges, servers, computing devices, storage devices. The network devices within the network 108 may interact with the system 102 through communication links.

As discussed above, the system 102 may be implemented in a computing device 104, such as a hand-held device, a laptop or other portable computer, a tablet computer, a mobile phone, a PDA, a smartphone, and a desktop computer. The system 102 may also be implemented in a workstation, a mainframe computer, a server, and a network server. In an embodiment, the system 102 may be coupled to a data repository, for example, a repository 112. The repository 112 may store data processed, received, and generated by the system 102. In an alternate embodiment, the system 102 may include the data repository 112.

The network environment 100 supports various connectivity options such as BLUETOOTH®, USB, ZigBee and other cellular services. The network environment enables connection of devices 106 such as Smartphone with the server 104, and accordingly with the database 112 using any communication link including Internet, WAN, MAN, and so on. In an exemplary embodiment, the system 102 is implemented to operate as a stand-alone device. In another embodiment, the system 102 may be implemented to work as a loosely coupled device to a smart computing environment.

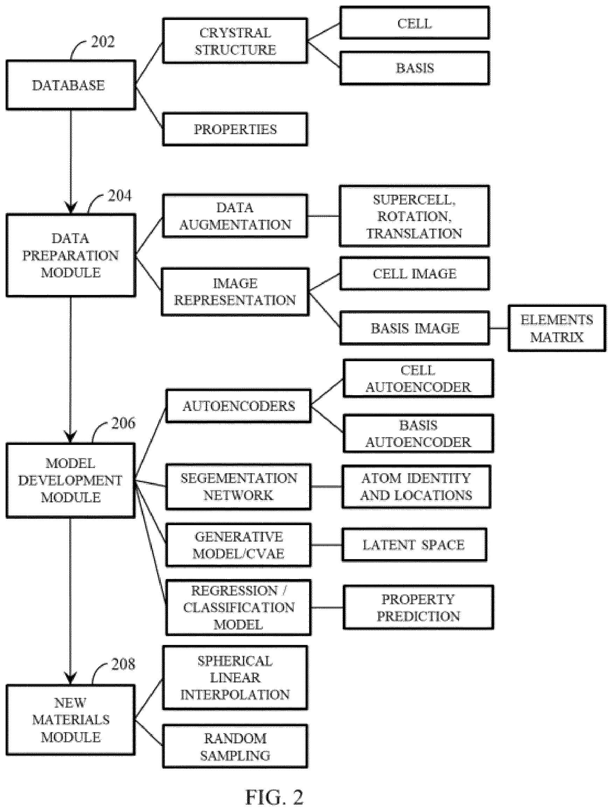

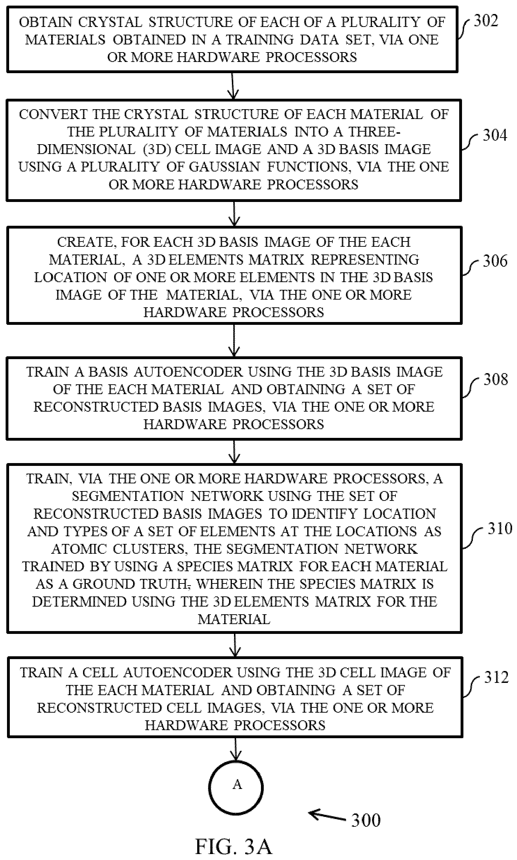

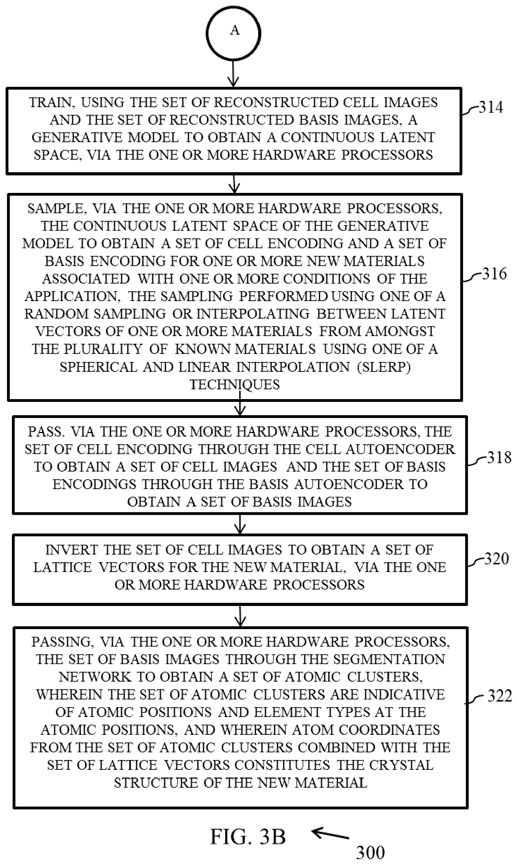

Referring to FIG. 2, an architectural overview of modules of the system 102 is illustrated in accordance with an example embodiment of the present disclosure. The architecture of the system 102 is shown to include a database 202, a data preparation module 204, a model development module 206, and a new materials designing module 208. The database 202 may be an example of the repository 112. The database 202 may store a training data required for the purpose of material design. The aforementioned components of the system 102 and functionalities thereof are explained further in detail with reference to FIGS. 2 and 3 collectively.

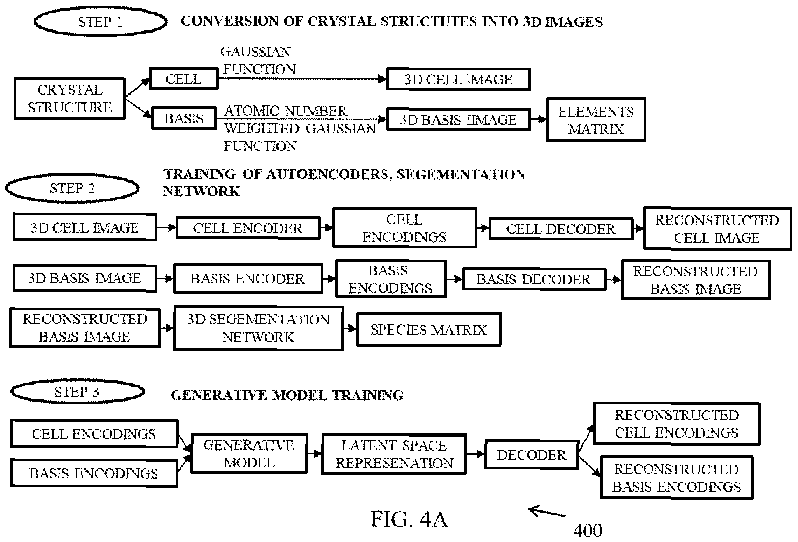

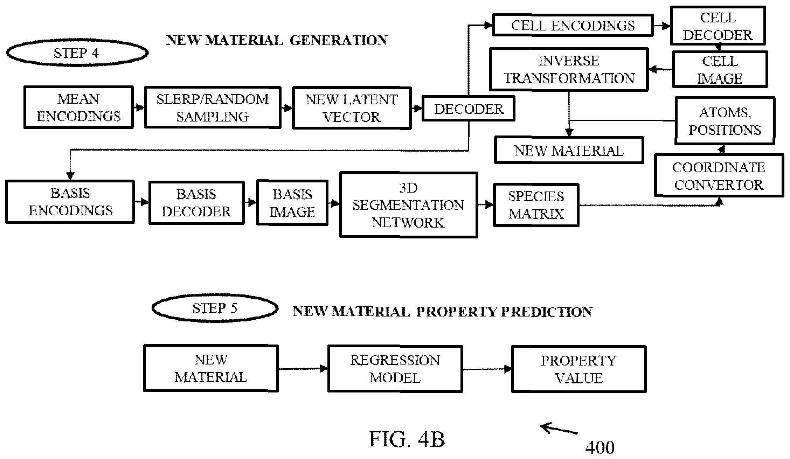

Referring collectively to FIGS. 2, 3 and 4A-4B, designing of functional materials for specific applications is described in accordance with various embodiments of the present disclosure. For example, FIG. 3 illustrates an example flow chart of a method 300 for application based designing of new functional materials, in accordance with an example embodiment of the present disclosure. FIGS. 4A-4B illustrate a flow-diagram of a method 400 for designing of functional materials for specific applications, in accordance with an example embodiment. The methods 300 and 400 depicted in the flow chart and flow-diagram respectively, may be executed by a system, for example, the system, 102 of FIG. 1. In an example embodiment, the system 102 may be embodied in a computing device.

Operations of the flowchart, and combinations of operation in the flowchart, may be implemented by various means, such as hardware, firmware, processor, circuitry and/or other device associated with execution of software including one or more computer program instructions. For example, one or more of the procedures described in various embodiments may be embodied by computer program instructions. In an example embodiment, the computer program instructions, which embody the procedures, described in various embodiments may be stored by at least one memory device of a system and executed by at least one processor in the system. Any such computer program instructions may be loaded onto a computer or other programmable system (for example, hardware) to produce a machine, such that the resulting computer or other programmable system embody means for implementing the operations specified in the flowchart. It will be noted herein that the operations of the method 300/400 are described with help of system 102. However, the operations of the method 300/400 can be described and/or practiced by using any other system.

The system 102 is configured to train models for designing and discovery of materials with application based property. The training data set may include crystal structures and properties of a plurality of known materials. In an embodiment, the training data set may be stored in the repository, for example, the repository 112 of the system 102. In an embodiment, crystal structure of each of the plurality of materials in the training data set may be obtained at 302.

The data preparation module 204 of the system 102 facilitates in cleaning of the training data, data augmentation and creation of 3D images from crystallographic data. The data augmentation includes, for example, supercell construction, rotation, and translation. After creating the augmented dataset, 3D cell and basis images are constructed for each of the crystal structure by mapping the lattice and basis to a cubic grid. For example, at 304 of the method 300, the crystal structure of each material of the plurality of materials are converted into a three-dimensional (3D) cell image and a 3D basis image using a plurality of gaussian functions. For instance, the lattice of a materials' crystal structure may be converted to a 3D cell image using a Gaussian function, and the atomic basis may be converted to a 3D basis image using an atomic number weighted Gaussian function. Following data preparation, the 3D cell images and 3D bases images are utilized for training the autoencoders and a segmentation network.

At 306, the method 300 includes creating, for each 3D basis image of each of the materials, a 3D elements matrix representing location of one or more elements in the 3D basis image of the material. A basis autoencoder is trained using the 3D basis image of the each material and a set of reconstructed basis images are obtained at 308.

Following training of the basis autoencoder, the segmentation network may be trained using the reconstructed basis images (i.e., images obtained as the output from the decoder of the basis autoencoder) to identify location and types of elements at that location as atomic clusters. At 310, the method 300 includes training a segmentation network using the set of reconstructed basis images to identify location and types of a set of elements at the locations as atomic clusters. The segmentation network is trained by using a species matrix for each material as the ground truth. The species matrix is determined using the 3D elements matrix for the material. In an embodiment, the segmentation network may be a 3D attention U-net model.

At 312, the method 300 includes training a cell autoencoder using the 3D cell image of the each material and obtaining a set of reconstructed cell images. In an embodiment, the cell autoencoder and the basis autoencoder may be built as 3D convolutional neural nets (3D CNNs). In an example, embodiment, a mean squared error (MSE), defined below, may be used as the loss function to train the basis autoencoder and the cell autoencoder.

$$MSE = \frac{1}{n * D} \sum_{i=1}^{n} (Y_i - \widehat{Y_i})^2$$

where 'n' is the number of images (or training data points), $Y_i$ is the ground truth, $\hat{Y_i}$ is the value predicted by the autoencoder, and 'D' is the dimensionality of image.

At 314, the method 300 includes training a generative model using the set of reconstructed cell images and the set of reconstructed basis images to obtain a continuous latent space. At 316, the method 300 includes sampling the continuous latent space of the generative model to obtain a set of cell encoding and a set of basis encoding for one or more new materials associated with one or more conditions of the application, the sampling performed using, for example, a random sampling or interpolating between latent vectors of one or more materials from amongst the plurality of known materials using, for example, a spherical or linear interpolation (SLERP) techniques;

At 318, the method 300 includes passing the set of cell encoding through the cell autoencoder to obtain a set of sampled cell images and the set of basis encodings through the basis autoencoder to obtain a set of sampled basis images. At 320, the method 300 includes inverting the set of sampled cell images to obtain a set of lattice vectors for the one or more new materials. At 322, the method 300 includes passing the set of sampled basis images through the segmentation network to obtain a set of atomic clusters. The set of atomic clusters are indicative of atomic positions and element types at the atomic positions. The atom coordinates from the set of atomic clusters combined with the set of lattice vectors constitutes the crystal structure of the one or more new material.

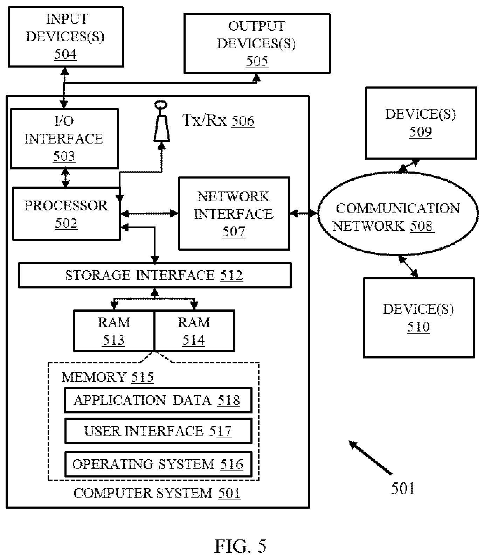

FIG. 5 is a block diagram of an exemplary computer system 501 for implementing embodiments consistent with the present disclosure. The computer system 501 may be implemented in alone or in combination of components of the system 102 (FIG. 1). Variations of computer system 501 may be used for implementing the devices included in this disclosure. Computer system 501 may comprise a central processing unit ("CPU" or "hardware processor") 502. The hardware processor 502 may comprise at least one data processor for executing program components for executing user- or system-generated requests. The processor may include specialized processing units such as integrated system (bus) controllers, memory management control units, floating point units, graphics processing units, digital signal processing units, etc. The processor may include a microprocessor, such as AMD AthlonTM, DuronTM or OpteronTM, ARM's application, embedded or secure processors, IBM PowerPCTM, Intel's Core, ItaniumTM, XeonTM, CeleronTM or other line of processors, etc. The processor 502 may be implemented using mainframe, distributed processor, multi-core, parallel, grid, or other architectures. Some embodiments may utilize embedded technologies like application specific integrated circuits (ASICs), digital signal processors (DSPs), Field Programmable Gate Arrays (FPGAs), etc. The processor 502 may be a multi-core multi-threaded processor.

Processor 502 may be disposed in communication with one or more input/output (I/O) devices via I/O interface 503. The I/O interface 503 may employ communication protocols/methods such as, without limitation, audio, analog, digital, monoaural, RCA, stereo, IEEE-1394, serial bus, universal serial bus (USB), infrared, PS/2, BNC, coaxial, component, composite, digital visual interface (DVI), high-definition multimedia interface (HDMI), RF antennas, S-Video, VGA, IEEE 802.11 a/b/g/n/x, Bluetooth, cellular (e.g., code-division multiple access (CDMA), high-speed packet access (HSPA+), global system for mobile communications (GSM), long-term evolution (LTE), WiMax, or the like), etc.

Using the I/O interface 503, the computer system 501 may communicate with one or more I/O devices. For example, the input device 504 may be an antenna, keyboard, mouse, joystick, (infrared) remote control, camera, card reader, fax machine, dongle, biometric reader, microphone, touch screen, touchpad, trackball, sensor (e.g., accelerometer, light sensor, GPS, gyroscope, proximity sensor, or the like), stylus, scanner, storage device, transceiver, video device/source, visors, etc.

Output device 505 may be a printer, fax machine, video display (e.g., cathode ray tube (CRT), liquid crystal display (LCD), light-emitting diode (LED), plasma, or the like), audio speaker, etc. In some embodiments, a transceiver 306 may be disposed in connection with the processor 502. The transceiver may facilitate various types of wireless transmission or reception. For example, the transceiver may include an antenna operatively connected to a transceiver chip (e.g., Texas Instruments WiLink WL1283, Broadcom BCM4750IUB8, Infineon Technologies X-Gold 618-PMB9800, or the like), providing IEEE 802.11a/b/g/n, Bluetooth, FM, global positioning system (GPS), 2G/3G HSDPA/HSUPA communications, etc.

In some embodiments, the processor 502 may be disposed in communication with a communication network 508 via a network interface 507. The network interface 507 may communicate with the communication network 508. The network interface may employ connection protocols including, without limitation, direct connect, Ethernet (e.g., twisted pair 10/100/1000 Base T), transmission control protocol/internet protocol (TCP/IP), token ring, IEEE 802.11a/b/g/n/x, etc. The communication network 308 may include, without limitation, a direct interconnection, local area network (LAN), wide area network (WAN), wireless network (e.g., using Wireless Application Protocol), the Internet, etc. Using the network interface 507 and the communication network 508, the computer system 501 may communicate with devices 509 and 510. These devices may include, without limitation, personal computer(s), server(s), fax machines, printers, scanners, various mobile devices such as cellular telephones, smartphones (e.g., Apple iPhone, Blackberry, Android-based phones, etc.), tablet computers, eBook readers (Amazon Kindle, Nook, etc.), laptop computers, notebooks, gaming consoles (Microsoft Xbox, Nintendo DS, Sony PlayStation, etc.), or the like. In some embodiments, the computer system 501 may itself embody one or more of these devices.

In some embodiments, the processor 502 may be disposed in communication with one or more memory devices (e.g., RAM 513, ROM 514, etc.) via a storage interface 512. The storage interface may connect to memory devices including, without limitation, memory drives, removable disc drives, etc., employing connection protocols such as serial advanced technology attachment (SATA), integrated drive electronics (IDE), IEEE-1394, universal serial bus (USB), fiber channel, small computer systems interface (SCSI), etc. The memory drives may further include a drum, magnetic disc drive, magneto-optical drive, optical drive, redundant array of independent discs (RAID), solid-state memory devices, solid-state drives, etc. Variations of memory devices may be used for implementing, for example, any databases utilized in this disclosure.

The memory devices may store a collection of programs or database components, including, without limitation, an operating system 516, user interface application 517, user/application data 518 (e.g., any data variables or data records discussed in this disclosure), etc. The operating system 516 may facilitate resource management and operation of the computer system 501. Examples of operating systems include, without limitation, Apple Macintosh OS X, Unix, Unix-like system distributions (e.g., Berkeley Software Distribution (BSD), FreeBSD, NetBSD, OpenBSD, etc.), Linux distributions (e.g., Red Hat, Ubuntu, Kubuntu, etc.), IBM OS/2, Microsoft Windows (XP, Vista/7/8, etc.), Apple iOS, Google Android, Blackberry OS, or the like. User interface 517 may facilitate display, execution, interaction, manipulation, or operation of program components through textual or graphical facilities. For example, user interfaces may provide computer interaction interface elements on a display system operatively connected to the computer system 501, such as cursors, icons, check boxes, menus, scrollers, windows, widgets, etc. Graphical user interfaces (GUIs) may be employed, including, without limitation, Apple Macintosh operating systems' Aqua, IBM OS/2, Microsoft Windows (e.g., Aero, Metro, etc.), Unix X-Windows, web interface libraries (e.g., ActiveX, Java, Javascript, AJAX, HTML, Adobe Flash, etc.), or the like.

In some embodiments, computer system 501 may store user/application data 318, such as the data, variables, records, etc. as described in this disclosure. Such databases may be implemented as fault-tolerant, relational, scalable, secure databases such as Oracle or Sybase. Alternatively, such databases may be implemented using standardized data structures, such as an array, hash, linked list, structured text file (e.g., XML), table, or as hand-oriented databases (e.g., using HandStore, Poet, Zope, etc.). Such databases may be consolidated or distributed, sometimes among various computer systems discussed above. It is to be understood that the structure and operation of any computer or database component may be combined, consolidated, or distributed in any working combination.

Additionally, in some embodiments, (the server, messaging and instructions transmitted or received may emanate from hardware, including operating system, and program code (i.e., application code) residing in a cloud implementation. Further, it should be noted that one or more of the systems and methods provided herein may be suitable for cloud-based implementation. For example, in some embodiments, some or all of the data used in the disclosed methods may be sourced from or stored on any cloud computing platform.

Example scenario:

An example scenario illustrating discovery of two-dimensional (2D) materials as photo-catalysts for water splitting is provided. In the presented example, the target was to generate new 2D materials with appropriately aligned band edges to facilitate water splitting reaction.

The data for training the models consisted of first principles computed structures and bandgaps of 2D materials, taken from openly available 2D materials databases and curated to remove multiple entries with the same structure. Subsequently, all the slabs were translated such that the center of the 2D material along the surface normal direction, lay at the center of the periodic box. Since the number of datapoints in the disclosed materials database was only a few thousands, the available data was augmented by creating supercells as well as applying random translations and rotations to the structure of these materials. After creating the augmented dataset, 3D cell and basis images were constructed for each of the crystal structure by mapping the lattice and basis to a cubic grid. All these images had a dimension of (32x32x32). The voxel values for the 3D cell images were obtained from the lattice parameters of the crystal using:

$$F(i,j,k) = A*exp(-rijk^2/2\sigma^2)$$

where rijk is the distance between the (i,j,k) voxel and center of the lattice, $\sigma$ is the gaussian width and A is a pre-factor.

For constructing the basis image, the atoms in the crystal were first translated into a cube of edge 10 Å such that the center of the basis coincided with that of the cube. The voxel values of the (32x32x32) basis image were computed using

$$G(i,j,k) = \frac{1}{\sigma^3 (2\pi)^{1.5}} \sum_l Z_l exp\left(-\frac{d(Z_l,(i,j,k))^2}{2\sigma^2}\right)$$

$Z_l$ was the atomic number of atom 'l' in the basis, $d(Z_l,(i,j,k))$ was the distance between the (i,j,k) voxel and the coordinates of atom 'l' and $\sigma$ was the gaussian width.

Following basis image creation, the elements matrix was constructed as a (32x32x32) matrix using:

$$S(i,j,k) = Z_l \text{ if } d(Z_l,(i,j,k)) < 0.5 \text{ Å; else } S(i,j,k) = 0$$

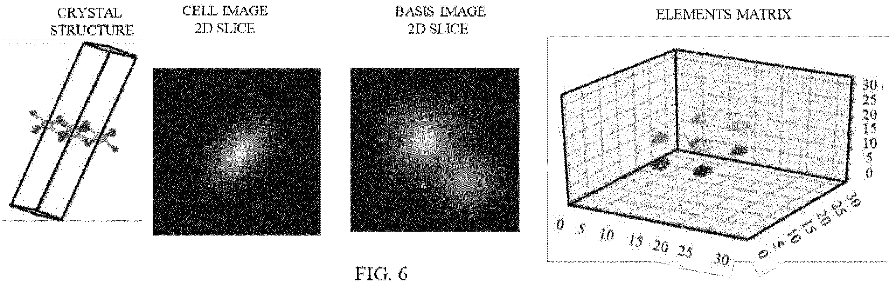

FIG. 6 shows a pictorial representation of the cell and basis images, species matrix for a 2D material in the training dataset.

Following data preparation, the cell and basis autoencoders were trained using the respective 3D images. Both the autoencoders were built as 3D convolutional neural nets (3D CNNs). The encoder of the cell autoencoder consisted of four 3D convolutional layers while the decoder used four 3D convolution transpose layers (i.e., a mirror image of the encoder). Similarly, the encoder of the basis autoencoder consisted of four 3D convolutional layers. However, the decoder used upsampling instead of 3D convolution transpose. The dimensions of cell and basis encoding vectors (i.e., the autoencoder bottleneck dimension) were 128 and 256 respectively. A detailed description of the successive layers in the encoder and decoder of the cell and basis autoencoders are given below in tables A, B, and C.

Cell autoencoder consisted of an encoder and a decoder

Table A

| Cell autoencoder |
| --- |
| **Encoder** |
| Convolution 3D, kernel Size=4, strides =2, channels=64, padding='same', activation=LeakyReLU(alpha=0.2) |
| Convolution 3D, kernel Size=4, strides =2, channels=64, padding='same', activation=LeakyReLU(alpha=0.2) |
| Convolution 3D, kernel Size=4, strides =2, channels=64, padding='same', activation=LeakyReLU(alpha=0.2) |
| Convolution 3D, kernel Size=4, strides =1, channels=128, padding='valid', activation=tanh |

Table B

| Cell autoencoder |
| --- |
| **Decoder** |
| Convolution 3DTranspose, kernel size=4, strides=1, channels=64,padding='valid', activation=LeakyReLU (alpha=0.2) |
| Convolution 3Dtranspose, kernel size=4, strides=2,channels=64,padding='same', activation=LeakyReLU (alpha=0.2) |
| Convolution 3Dtranspose, kernel size=4, strides=2, channels=64,padding='same' , activation=LeakyReLU (alpha=0.2) |
| Convolution 3Dtranspose, kernel size=4, strides=2,channels=1,padding='same', activation=sigmoid |
| Lambda layer: x=clipping output of last layer in [0.5, 0.5001], Take minimum of ((x-0.5) * 10000, 1) and passed as output |

Table C

| Basis Autoencoder |
| --- |
| **Encoder** |
| Convolution 3D, kernel Size=5, strides =2, channels=16, padding='valid' |
| Batch Normalization, activation=LeakyReLU(alpha=0.3) |
| Convolution 3D, kernel Size=3, strides =1, channels=32, padding='valid' |
| Batch Normalization, activation=LeakyReLU(alpha=0.3) |
| Convolution 3D, kernel Size=3, strides =1, channels=64, padding='valid' |
| Batch Normalization, activation=LeakyReLU(alpha=0.3) |
| Convolution 3D, kernel Size=3, strides =2, channels=128, padding='valid' |
| Batch Normalization, activation=LeakyReLU(alpha=0.3) |
| Fully connected layer, size=256 |

(continued)

| Decoder |
| --- |
| Fully connected layer, size=5*5*5*128 |
| Reshape to 5,5,5,128 |
| UpSampling 3D, factor=2 |
| Replication Padding 3D, padding=(2,2,2) |
| Convolution 3D, kernel Size=5, strides =1, channels=64, padding='valid' |
| Batch Normalization, activation=LeakyReLU(alpha=0.3) |
| UpSampling 3D, factor=2 |
| Replication Padding 3D, padding=(1,1,1) |
| Convolution 3D, kernel Size=5, strides =1, channels=32, padding='valid' |
| Batch Normalization, activation=LeakyReLU(alpha=0.3) |
| UpSampling 3D, factor=2 |
| Replication Padding 3D, padding=(1,1,1) |
| Convolution 3D, kernel Size=4, strides =1, channels=16, padding='valid' |
| Batch Normalization, activation=LeakyReLU(alpha=0.3) |
| Convolution 3D, kernel Size=4, strides =1, channels=1, padding='valid', activation=ReLU |

Mean squared error (MSE), defined below, was used as the loss function to train both basis and cell autoencoders.

$$MSE = \frac{1}{n * D} \sum_{i=1}^{n} (Y_i - \widehat{Y_i})^2$$

where '$n$' is the number of images (or training data points), 'D' is the dimensionality of an image, $Y_i$ is the ground truth and $\hat{Y_i}$ is the value predicted by the autoencoder.

Following training of the basis autoencoder, the segmentation network (a 3D attention U-net model) was trained using the reconstructed basis images (i.e., images obtained as the output from the decoder of the basis autoencoder) to identify location and types of elements at that location as atomic clusters. The elements matrix prepared earlier for each structure was converted into a species matrix via one hot encoding into 95 classes at each grid point. Of these 95 classes, one class corresponded to the background (or vacuum) while the other 94 classes corresponded to different elements. If a particular element type was present at a grid point of the elements matrix, its corresponding class was set to 1 while the rest of the values of the one hot vector remained as zeros. Thus, for each material, the ground truth to train the segmentation network was a species matrix of dimension (32x32x32x95). The binary cross entropy (BCE) loss was used while training the segmentation network.

The next step involved training a generative model to obtain a continuous latent space that can be sampled to obtain new materials. For a material to qualify as a photocatalyst, two of the necessary conditions are that the material must be thermodynamically stable and semiconducting in nature. As a thumb rule, a material was considered stable if its energy above the hull (e_hull) value was less than 150meV per atom. Thus, the training data had materials belonging to four different classes: (i) Stable and nonmetal (i.e, e_hull < 150meV and band gap > 0), (ii) Stable and metal (i.e., e_hull < 150meV and band gap = 0), (iii) Unstable and nonmetal (i.e., e_hull > 150meV and band gap > 0) and (iv) Unstable and metal (i.e., e_hull > 150meV and bandgap = 0). The aim in generative modeling was to sample the latent space to obtain materials belonging to class (i), so that new 2D photocatalysts can be identified. While a number of different generative modeling techniques exist, in the present case study, we chose a Conditional Variational Auto Encoder (CVAE) as the disclosed generative model so that while sampling the latent space for new materials, control can be exerted over the class of material to be generated (i.e., material belonging to class (i) described above). The training data was one hot encoded as shown below in table D:

Table D

| One hot encoding of materials based on their bang gap and e_hull values | | |
|---|---|---|
| Condition | One hot encoding | Category |
| (i) gap >0 eV, e-hull < 0.15 eV | [1,0,0,0] | Nonmetal, stable |
| (ii) gap = 0 eV, e-hull <0.15 eV | [0,1,0,0] | Metal, stable |
| (iii) gap >0 eV, e-hull >0.15 eV | [0,0,1,0] | Non-metal, unstable |
| (iv) gap =0 eV, e-hull>0.15 eV | [0,0,0,1] | Metal, unstable |

CVAE was trained using the cell and basis encodings from the previous step together with the one hot encoded vectors. Cell encodings were padded with zeros such that both the cell and basis encodings were 256-dimension vectors. Subsequently, these were scaled using the normal quantile transformer with 1000 quantiles. The four dimensional one-hot encoded vector was connected to a 256 dimension hidden layer so that the cell, basis and the class encodings were all 256 dimensional vectors. These vectors were then concatenated as 'channels' so that each training data was now represented by a (256x3) dimension image. CVAE network comprised of a probabilistic encoder and a probabilistic decoder. We represented both the encoder and the decoder via 2D CNNs. The detailed architecture of the disclosed CVAE model is given below in table E:

Table E

| CVAE architecture |
|---|
| Inputs : cell encoding vector : (1,256,1), basis encoding vector: (1,256,1), property: (4,) |
| **Encoder** |
| Fully-collected layer with property input vector, size=256; Reshape to (1, 256,1) |
| Concatenation with cell encoding, basis encodings along channels : (1,256,3) |
| Convolution 2D { kernel Size=3, strides =2, channels=16, padding='same'} , batch Normalization, activation=LeakyReLU(alpha=0.2) |
| Convolution 2D { kernel Size=3, strides =2, channels=32, padding='same'} , batch Normalization, activation=LeakyReLU(alpha=0.2) |
| Convolution 2D { kernel Size=3, strides =2, channels=64, padding='same'} , batch Normalization, activation=LeakyReLU(alpha=0.2) |
| Convolution 2D { kernel Size=3, strides =1, channels=128, padding='same'} , batch Normalization, activation=LeakyReLU(alpha=0.2) |
| Fully connected layer ( size= 512), batch Normalization, activation=LeakyReLU(alpha=0.2) |
| $\mu$ =Fully connected layer(size =128), log $\sigma^2$ = Fully connected layer(size =128) |
| |
| **Decoder** |
| Input : Sample latent vector shape =(128,), Property vector shape =(4,) |
| Concatenation to shape (132,) |
| Fully connected layer size= 4096, BatchNormalization, LeakyReLU, Reshape to (1,32,128) |
| Convolution 2D Transpose { kernel Size=3, strides =(1,1), channels=128, padding='same'} , batch Normalization, activation=LeakyReLU(alpha=0.2) |
| Convolution 2D Transpose { kernel Size=3, strides =(1,2) channels=64, padding='same'} , batch Normalization, activation=LeakyReLU(alpha=0.2) |
| Convolution 2D Transpose { kernel Size=3, strides =(1,2) channels=32, padding='same'} , batch Normalization, activation=LeakyReLU(alpha=0.2) |

(continued)

| Decoder |
| --- |
| Convolution 2D Transpose { kernel Size=3, strides =(1,2) channels=2, padding='same'} , activation=linear |

The probabilistic encoder encoded the input into a distribution with mean $\mu$ and standard deviation $\sigma$. A latent vector was then sampled from this distribution using the reparameterization trick, $z = \mu + \varepsilon * \sigma$, where $\varepsilon$ is random variable from normal distribution. This vector was passed through the probabilistic decoder to obtain the cell and basis encodings as the output. The loss function was defined as:

$$Loss = MSE_{rec} + \alpha * (KL\text{-}loss)$$

Where KL-loss was the Kullback-Leibler divergence term used to regularize the latent space to the prior distribution (which was a normal distribution with a mean 0 and standard deviation of unity) and $MSE_{rec}$ was mean square error in reconstruction of the basis and cell encodings.

After training CVAE model, a continuous latent space was obtained which could be sampled either randomly or by interpolating between the latent vectors of two known materials from the training data using techniques such as Spherical Linear Interpolation (SLERP). Furthermore, the newly generated material must belong to class (i) (i.e, thermodynamically stable and semiconducting) for it to be a potential photocatalyst. Thus, the sampled latent vector together with the one hot vector for class(i) was passed through the probabilistic decoder to obtain the cell and basis encodings. These encodings were then passed through the decoder of the cell and basis autoencoders to obtain the respective images. The cell image was inverse transformed to get the lattice parameters of the new material, namely, cell length and angles. The basis image was passed through the segmentation network to obtain the elements matrix, identifying the location of the elements in the (32x32x32) grid as well as the types of those elements at that location, which was then used to obtain the cartesian positions of atoms. This information was combined with the cell lengths and angles to obtain the newly generated 2D material.

In this case study, since the intended application of the discovered 2D photocatalysts was in water splitting, it is essential that the band gap and the band edges of the material are aligned appropriately. The bandgap of the generated material was predicted using the Crystal Graph Convolutional Neural Network (CGCNN) model whose weights were retrained using the bandgap of the 2D materials in the database. The band gap obtained from this model was used in an empirical equation, as given below, to obtain the location of the valence and conduction band edges.

$$E_{CB}^0 = \omega(X) - E_e - \frac{1}{2}E_g$$

$$E_{VB}^0 = \omega(X) - E_e + \frac{1}{2}E_g$$

$$\omega(X) = \sqrt[N]{X_1^a X_2^b X_3^c \dots X_n^q}$$

where $E_{CB}^0$ and $E_{VB}^0$ were the conduction and valence band edge energies, $E_g$ was the band gap predicted by the CGCNN model, $E_e$ was the absolute electrode potential of the standard hydrogen electrode and X was the electronegativity of the constituent elements in the material.

The above described networks were trained on a database of 2D materials that contained their structures and bandgaps. Instead of initializing the weights of the networks to random values, a more intelligent guess for these weights was obtained by pretraining these networks on the data from the Materials Project (MP) database (https://materialsproject.org). Only those materials from MP database were considered whose cell lengths were less than 10 Å, to ensure that accuracy of the model is not compromised due to coarseness of the grid resulting from use of larger cell lengths. It must be noted that the cell and basis were represented by (32x32x32) dimension images. Including materials with larger cell lengths would necessitate a finer image (say (64x64x64)) thereby increasing the memory footprint of the model. This however is not a restriction of the disclosed framework by any means and can be deployed effortlessly on hardware containing more memory per GPU card. The total curated crystal structure data from the MP database consisted of 54,727 materials which was randomly divided into train and test data in 90:10 ratio. The networks were pretrained on this data using the hyperparameters given below:

Cell autoencoder

Batch size =32, optimizer=Adam, initial learning rate =0.0001, Learning rate schedule= ReduceOnPlateau with patience of 20 epoch and factor of 0.5. Minimum leaning rate was set as 0.00001. Early stopping criteria was used with patience on 50 epochs.

Basis autoencoder

Batch size=32, optimizer=Adam, initial learning rate =0.0001, Learning rate scheduler = ReduceOnPlateau with patience of 20 epochs and factor=0.5. Minimum learning rate was set to 0.00001. Early stopping criteria was used with patience of 50 epochs

Segmentation network

Batch size=20, optimizer=Adam, initial learning rate=0.00005. Learning rate scheduler = ReduceOnPlateau with patience of 10 epochs and factor =0.5. minimum learning rate was set to 0.000025. Early stopping criteria was used with patience of 50 epochs.

CVAE model

Batch size=128, $\alpha$=0.1, initial learning rate=0.0001, total epochs=10,000, Learning rate scheduler=ReduceOnPlateau with patience of 100 epochs and factor =0.9. minimum learning rate is set as 0.00001

The weights after training the above networks were used as the initial guess to train the disclosed models for 2D materials. The curated dataset for 2D materials consisted of 6356 structures. This data was augmented by constructing supercells as well as random rotations and translations of the structures, resulting in an augmented dataset containing 0.2 million structures. The networks for the 2D materials were trained using this data, with a 90:10 training to test split. Following were the hyperparameters used in training the networks for the 2D materials:

Cell autoencoder

Batch size =64, optimizer=Adam, initial learning rate =0.0001, Learning rate schedule= ReduceOnPlateau with patience of 20 epoch and factor of 0.5. Minimum leaning rate was set as 0.00001. Early stopping criteria was used with patience on 50 epochs.

Basis autoencoder

Batch size=24, optimizer=Adam, initial learning rate =0.00001, Learning rate scheduler = ReduceOnPlateau with patience of 20 epochs and factor=0.5. Minimum learning rate was set to 0.000001. Early stopping criteria was used with patience of 100 epochs.

Segmentation network

Batch size=16, optimizer=Adam, initial learning rate=0.00001. Learning rate scheduler = ReduceOnPlateau with patience of 10 epochs and factor =0.5. Minimum learning rate was set to 0.000005. Early stopping criteria was used with patience of 50 epochs.

CVAE model

Batch size=128, $\alpha$=0.0001, initial learning rate=0.00005, total epochs=10,000, Learning rate scheduler = ReduceOnPlateau with patience of 100 epochs and factor =0.9. Minimum learning rate = 0.00001.

A summary of the test set errors in the cell and basis autoencoders, segmentation network is given in table 1. Note that the MSE and MAE for the cell and basis autoencoders correspond to the errors in reconstructing the input images to the outputs of the respective networks. Similarly, for the segmentation network, these values correspond to the errors in reproducing the species matrix.

**Table 1:** Summary of test error in various networks

|  | Mean Squared Error (MSE) | Mean Absolute Error (MAE) |
|---|---|---|
| Cell autoencoder | $3.17* 10^{-8}$ | $8.32*10^{-6}$ |
| Basis autoencoder | $1.99*10^{-4}$ | $6.59*10^{-3}$ |
|  | Binary cross entropy loss (BCE) | Mean Absolute Error (MAE) |
| Segmentation Network | $3.60*10^{-5}$ | $2.17*10^{-5}$ |

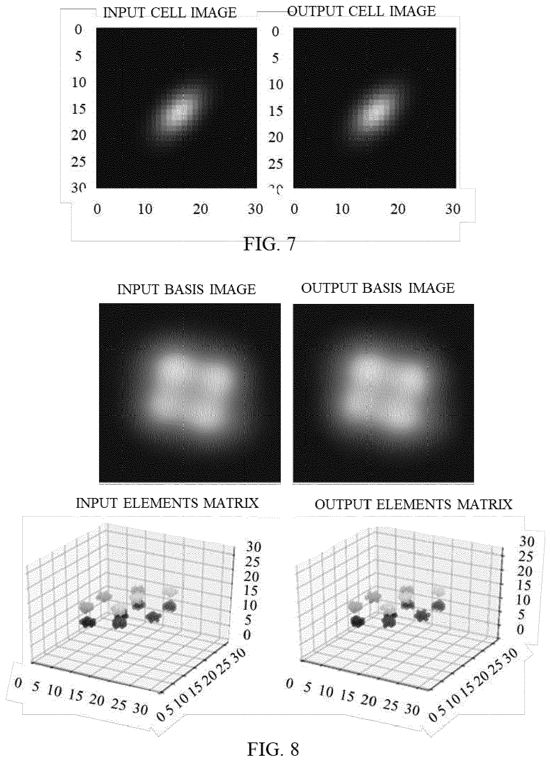

The cell parameters (i.e., the cell lengths and angles) were obtained from the output (i.e., the decoded cell image) of the cell autoencoder by feeding the voxel values to the inverse of the gaussian function that was used to construct the cell images originally. Firstly, it was observed that the intrinsic error (i.e., the error in transforming the lattice to cell image and back calculating the lattice parameters from the constructed image) in the cell image representation was zero, suggesting that the lattice to image transformation was perfect. Secondly, it was observed that the error in cell lengths and angles obtained upon inverting the output image from the cell autoencoder was also very small, suggesting that the cell autoencoder was able to learn the encodings very well. Table 2 shows the errors obtained upon reconstruction of the cell images while FIG. 7 shows a 2D slice of the input and reconstructed cell images for one of the materials in the test dataset.

**Table 2:** Reconstruction error in cell parameters

|  | a(Å) | b(Å) | c(Å) | $\alpha$(°) | $\beta$(°) | $\gamma$(°) |
|---|---|---|---|---|---|---|
| Intrinsic | 0.00 | 0.00 | 0.00 | 0.00 | 0.00 | 0.00 |
| Test set | 0.04 | 0.04 | 0.02 | 0.70 | 0.61 | 0.87 |

In comparison to cell, obtaining the atomic positions from the basis autoencoder and segmentation network was slightly more involved. To obtain error in atomic positions, the output of segmentation network was first converted to element matrix by using argmax function on one-hot encoded species matrix, to assign atomic numbers to each voxel position. To obtain the atoms and their position, first clusters were found in the elements matrix. Then, positions of the atoms were assigned as the centroid of clusters while the type of atom at that location (i.e. the atomic number) was assigned based on majority voting among voxels belonging to that cluster. The error in the atomic position was obtained by computing the distance between the predicted atom '$i$' in output element matrix and the nearest true atom '$j$' in the original element matrix (i.e., ground truth) of that material. The intrinsic error in atomic positions was found to be 0.051 Å. FIG. 8 shows a 2D slice of the input and output basis images as well as the corresponding input and output species matrix for a material from the test set.

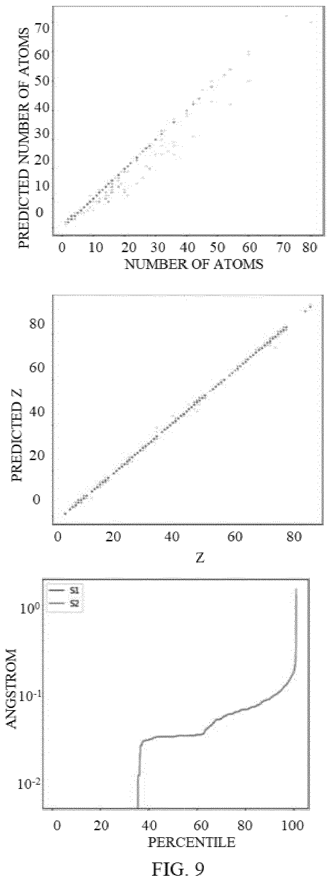

FIG. 9 shows the performance of segmentation network in terms of its predictions on the number of atoms in a material, types of these atoms and their positions for materials in the test set. It can be inferred that model was able to locate the atom position and atom types with great precision. Specifically, it was found that in 92.67 % of test data, the network identified correct number of atoms with an error of <= 0.06 Å in their location. When the condition on the error in the positions was relaxed to 0.5 Å, the model was able to identify the correct number of atoms with more than 99 % accuracy.

Finally, after training the cell, basis autoencoders and the segmentation network, the cell parameters obtained by inverting the output of the cell autoencoder and the atomic positions and types obtained from the basis autoencoder and the segmentation network were combined together to reconstruct the crystal structure of the original material. Errors in the cell parameters and the positions of the atoms after combining the two are given in table 3. Note that the error in the cell parameters in table 3 is different from that shown in table 2 since few materials were considered 'failed cases' as the segmentation network was unable to identify the correct number of atoms or atom types in these materials.

**Table 3:** Reconstruction errors in the crystal structure of materials in the test set.

|  | a(Å) | b(Å) | C(Å) | $\alpha$(°) | $\beta$(°) | $\gamma$(°) | Positions(Å) | Failed_ due_Z |
|---|---|---|---|---|---|---|---|---|
| Intrinsic | 0.00 | 0.00 | 0.00 | 0.0 | 0.0 | 0.0 | 0.04 | 1 |
| Reconstr uction | 0.03 | 0.04 | 0.022 | 0.67 | 0.60 | 0.80 | 0.053 | 93 |

Having trained the cell, basis autoencoders and the segmentation network, the generative model (CVAE) was trained next. Once again, pre-trained weights from the MP dataset was taken as the initial guess for the CVAE model. Table 4 lists the CVAE test set errors after training the model.

Table 4: Test set error at the end of CVAE model training

|  | KL loss | Reconstruction loss (MSE) |
| --- | --- | --- |
| CVAE | 4.79 | 0.012 |

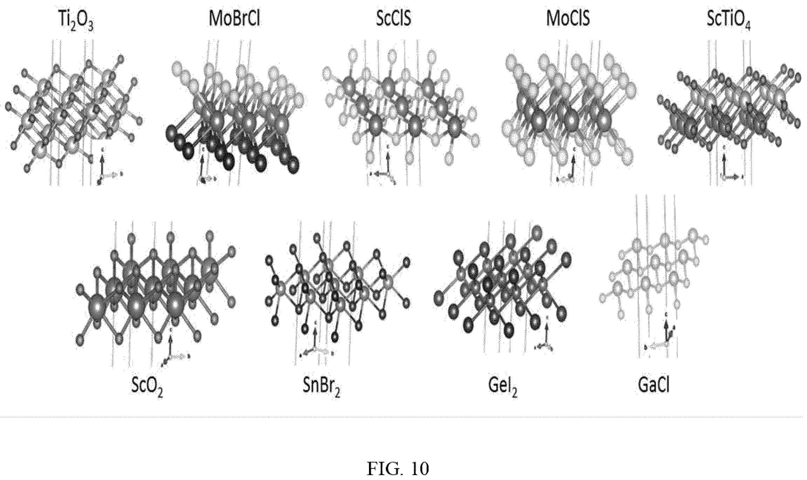

After training the CVAE model, the latent space was sampled to obtain cell and basis encodings for materials conditional on class (i) (i.e., thermodynamically stable and non-metal), via spherical linear interpolation between the latent vectors of two known 2D materials that served as the end points for interpolation. The trained CGCNN model was used to predict the band gap of these materials while the empirical equations described above were used to obtain the positions of the band edges. For a potentially good photocatalyst for water splitting, the band gap of the material must be between ~1.6 eV and ~3eV so that much of the energy in the solar spectrum can be absorbed to create charge carriers. Furthermore, the conduction band edge should lie below 0eV so that the electrons that populate the conduction band upon photoexcitation lie at a more negative potential than the standard reduction potential for the $H^+/H_2$ couple (which is 0V by definition for SHE) while the valence band edge should lie above 1.23 eV so that the holes created in the valence band upon electron excitation lie at a more positive potential than the oxidation potential for the $H_2O/O_2$ couple (=1.23V vs SHE). Nine different materials were found (by interpolating between the latent vectors of different known materials) that had band gaps and band edges ideally located for photocatalytic water splitting. Table 5 lists these materials, their band gaps and band edge positions while FIG. 10 shows their predicted crystal structures.

Table 5: 2D materials for photocatalytic water splitting generated by sampling the continuous latent space of the CVAE model. CBM and VBM correspond to conduction band minimum and valence band maximum respectively

| Generated material | New / known material | Band Gap(eV) | CBM(eV) | VBM(eV) |
| --- | --- | --- | --- | --- |
| Ti2O3 | New | 3.3 | -0.9 | 2.4 |
| MoBrCl | New | 2.7 | -0.6 | 2.1 |
| MoClS | New | 1.6 | -0.3 | 1.3 |
| ScTiO4 | New | 3.0 | -0.5 | 2.5 |
| ScClS | New | 2.2 | -0.7 | 1.5 |
| ScO2 | known (in database) | 2.7 | -0.4 | 2.3 |
| SnBr2 | known (in database) | 2.0 | -0.4 | 1.6 |
| GeI2 | known (in database) | 2.0 | -0.6 | 1.4 |
| GaCl | known (in database) | 2.6 | -1.3 | 1.3 |

Of the nine materials, four of them were already known and present in the disclosed 2D material database, which validates the ability of the disclosed framework to generate realistic materials. Comparison of the bandgaps and the structural features of these materials, shown in table 6, further attests to the reliability of the disclosed model.

Table 6: Comparison of the bandgaps and structural parameters of the 'known' materials with their respective values in the 2D materials database.

|  | $\Delta$gap(eV) | $\Delta$a(Å) | $\Delta$b(Å) | $\Delta\alpha$(°) | $\Delta\beta$(°) | $\Delta\gamma$(°) |
| --- | --- | --- | --- | --- | --- | --- |
| ScO2 | 1.27 | -0.34 | -0.34 | -1.45 | -0.98 | 0.91 |
| SnBr2 | -0.39 | -0.22 | -0.22 | -1.09 | -0.65 | -0.71 |
| GeI2 | -0.13 | -0.08 | 0.34 | 1.77 | 2.96 | -1.2 |
| GaCl* | -0.43 | -2.38 | -2.36 | 0.37 | -0.21 | 29.75 |
| *The structure of GaCl predicted by the disclosed model was different from that present in the 2D materials database. | | | | | | |

Although only nine materials are reported here, far many more suitable materials can be obtained by drawing many samples from the continuous latent space of the CVAE model. A further validation/screening of these materials can then be performed using density functional theory calculations to narrow down the number of promising candidates to a few tens.

The written description describes the subject matter herein to enable any person skilled in the art to make and use the embodiments. The scope of the subject matter embodiments is defined by the claims.

Various embodiments describe method and system for design and discovery of functional materials based on the application property value. Conventional methods for the discovery of novel functional materials used laborious experimentation or costly first principles calculations. Some prior arts based data driven techniques for design of novel functional materials use point cloud-based representation for crystal structures. However, this representation suffers from permutation variance. Since permutation invariance is not inbuilt in a material's representation, the DL model has to learn this invariance which may not be accurate. Some other prior art uses image based representation for crystal structures. However, while representing the basis, they used separate images for each element type. This leads to huge memory requirement and also increases the training time many-fold. In addition to that, since each element is represented by its own image, it is difficult for the model to learn the chemical environment and neighborhood pattern of each element. The disclosed embodiments overcome the aforementioned limitations of the prior art by using an image based representation of materials consistent with physical principles. Specifically, just as a material is construed as a basis of atoms in a lattice, each crystal structure is represented using a cell and a basis image. In addition, the disclosed embodiments utilize elements matrix which facilitates in obtaining the atoms and their positions from basis images. Thus, any material, irrespective of the geometry of the lattice, the number and types of elements in the structure, is represented by only two images.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1.   A hardware processor (502) implemented method (300), comprising:

obtaining (302) a crystal structure of each material of a plurality of known materials from a training data set;
converting (304) the crystal structure of each material of the plurality of known materials into a three-dimensional -3D-cell image and a 3D basis image using a plurality of gaussian functions;
creating (306), for each 3D basis image of each material, a 3D elements matrix representing location of one or more elements in the 3D basis image of each material;
training (308) a basis autoencoder using the 3D basis image of each material and obtaining a set of reconstructed basis images:

training (310) a segmentation network using the set of reconstructed basis images to identify a location and types of a set of elements at that location as atomic clusters, wherein the segmentation network is trained by using a species matrix for each material as a ground truth, wherein the species matrix is determined using the 3D elements matrix for each material;
training (312) a cell autoencoder using the 3D cell image of each material and obtaining a set of reconstructed cell images;
training (314), using the set of reconstructed cell images and the set of reconstructed basis images, a generative model to obtain a continuous latent space;
sampling (316) the continuous latent space of the generative model to obtain a set of cells encoding and a set of basis encoding for one or more new materials, wherein the sampling is performed using one of a random sampling and interpolating between latent vectors of one or more materials from the plurality of known materials using one of a spherical and linear interpolation -SLERP-techniques;
passing (318) the set of cells encoding through the cell autoencoder to obtain a set of sampled cell images and the set of basis encodings through the basis autoencoder to obtain a set of sampled basis images;
inverting (320) the set of sampled cell images to obtain a set of lattice vectors for the one or more new materials;
passing (322) the set of sampled basis images through the segmentation network to obtain a set of atomic clusters, wherein the set of atomic clusters are indicative of atomic positions and element types at the atomic positions, and wherein atom coordinates from the set of atomic clusters combined with the set of lattice vectors constitutes a crystal structure of the one or more new materials;
training a regression model for prediction of target properties of the one or more new materials, wherein the continuous latent space of the generative model comprises features for the regression model; and
predicting the target properties of the one or more new materials based on the crystal structure of the one or more new materials.

2.   The processor implemented method of claim 1, wherein the training dataset comprises first principles computed structures and properties of the plurality of known materials.

3.   The processor implemented method of claim 2, wherein the training dataset is preprocessed, and wherein preprocessing the training dataset comprises:

removing redundant entries having same structure from the training dataset; and
augmenting the dataset by creating supercells and applying random translations and rotations to the structure of the plurality of known materials.

4.   The processor implemented method of claim 1, wherein obtaining the cells and basis encodings of the one or more new material comprises:

obtaining one or more latent vectors based on the sampling; and
obtaining the cells and basis encodings of the one or more new material by passing the one or more latent vectors through the generative model.

**5.** A system (500), comprising:

a memory (515) storing instructions;
one or more communication interfaces (503); and
one or more hardware processors (502) coupled to the memory (515) via the one or more communication interfaces (503), wherein the one or more hardware processors (502) are configured by the instructions to:

obtain a crystal structure of each material of a plurality of known materials from a training data set;
convert the crystal structure of each material of the plurality of known materials into a three-dimensional -3D-cell image and a 3D basis image using a plurality of gaussian functions;
create, for each 3D basis image of each material, a 3D elements matrix representing location of one or more elements in the 3D basis image of each material;
train a basis autoencoder using the 3D basis image of each material and obtaining a set of reconstructed basis images;
train a segmentation network using the set of reconstructed basis images to identify a location and types of a set of elements at that location as atomic clusters, wherein the segmentation network is trained by using a species matrix for each material as a ground truth wherein the species matrix is determined using the 3D elements matrix for each material;
train a cell autoencoder using the 3D cell image of each material and obtaining a set of reconstructed cell images;
train, using the set of reconstructed cell images and the set of reconstructed basis images, a generative model to obtain a continuous latent space;
sample the continuous latent space of the generative model to obtain a set of cells encoding and a set of basis encoding for one or more new materials, wherein the sampling is performed using one of a random sampling and interpolating between latent vectors of one or more materials from the plurality of known materials using one of a spherical and linear interpolation -SLERP- techniques;
pass the set of cells encoding through the cell autoencoder to obtain a set of sampled cell images and the set of basis encodings through the basis autoencoder to obtain a set of sampled basis images;
invert the set of sampled cell images to obtain a set of lattice vectors for the one or more new materials;
pass the set of sampled basis images through the segmentation network to obtain a set of atomic clusters, wherein the set of atomic clusters are indicative of atomic positions and element types at the atomic positions, and wherein atom coordinates from the set of atomic clusters combined with the set of lattice vectors constitutes a crystal structure of the one or more new materials;
train a regression model for prediction of target properties of the one or more new materials, wherein the continuous latent space of the generative model comprises features for the regression model; and
predict the target properties of the one or more new materials based on the crystal structure of the one or more new materials.

**6.** The system of claim 5, wherein the training dataset comprises first principles computed structures and properties of the plurality of known materials.

**7.** The system of claim 6, wherein the training dataset is preprocessed, and wherein to preprocess the training dataset, the one or more hardware processors are configured by the instructions to:

remove redundant entries having same structure from the training dataset; and
augment the dataset by creating supercells and applying random translations and rotations to the structure of the plurality of known materials.

**8.** The system of claim 5, wherein to obtaining the cells and basis encodings of the one or more new materials, the one or more hardware processors are configured by the instructions to:

obtain one or more latent vectors based on the sampling; and
obtain the cells and basis encodings of the one or more new material by passing the one or more latent vectors through the generative model.

**9.** One or more non-transitory machine-readable information storage mediums comprising one or more instructions which when executed by one or more hardware processors cause, by the one or more hardware processors (502):

obtaining a crystal structure of each material of a plurality of known materials from a training data set;

converting the crystal structure of each material of the plurality of known materials into a three-dimensional cell image and a 3D basis image using a plurality of gaussian functions;

creating, for each 3D basis image of each material, a 3D elements matrix representing location of one or more elements in the 3D basis image of each material;

training a basis autoencoder using the 3D basis image of each material and obtaining a set of reconstructed basis images;

training a segmentation network using the set of reconstructed basis images to identify a location and types of a set of elements at that location as atomic clusters, wherein the segmentation network is trained by using a species matrix for each material as a ground truth wherein the species matrix is determined using the 3D elements matrix for each material;

training a cell autoencoder using the 3D cell image of each material and obtaining a set of reconstructed cell images;

training, using the set of reconstructed cell images and the set of reconstructed basis images, a generative model to obtain a continuous latent space;

sampling the continuous latent space of the generative model to obtain a set of cells encoding and a set of basis encoding for one or more new materials, wherein the sampling is performed using one of a random sampling and interpolating between latent vectors of one or more materials from the plurality of known materials using one of a spherical and linear interpolation -SLERP- techniques;

passing the set of cells encoding through the cell autoencoder to obtain a set of sampled cell images and the set of basis encodings through the basis autoencoder to obtain a set of sampled basis images;

inverting the set of sampled cell images to obtain a set of lattice vectors for the one or more new materials; and passing the set of sampled basis images through the segmentation network to obtain a set of atomic clusters, wherein the set of atomic clusters are indicative of atomic positions and element types at the atomic positions, and wherein atom coordinates from the set of atomic clusters combined with the set of lattice vectors constitutes a crystal structure of the one or more new materials;

training a regression model for prediction of target properties of the one or more new materials, wherein the continuous latent space of the generative model comprises features for the regression model; and

predicting the target properties of the one or more new materials based on the crystal structure of the one or more new materials.

**Patentansprüche**

1. Hardware-Prozessor (502), implementiertes Verfahren (300), umfassend:

Erhalten (302) einer Kristallstruktur jedes Materials einer Mehrzahl von bekannten Materialien aus einem Trainingsdatensatz;

Umwandeln (304) der Kristallstruktur jedes Materials der Mehrzahl von bekannten Materialien in ein dreidimensionales 3D-Zellenbild und ein 3D-Basisbild unter Verwendung einer Mehrzahl von Gaußschen Funktionen;

Erstellen (306), für jedes 3D-Basisbild jedes Materials, einer 3D-Elementmatrix, die den Ort eines oder mehrerer Elemente in dem 3D-Basisbild jedes Materials darstellt;

Trainieren (308) eines Basis-Autoencoders unter Verwendung des 3D-Basisbildes jedes Materials und Erhalten eines Satzes von rekonstruierten Basisbildern;

Trainieren (310) eines Segmentierungsnetzwerks unter Verwendung des Satzes von rekonstruierten Basisbildern, um einen Ort und Typen eines Satzes von Elementen an diesem Ort als atomare Cluster zu identifizieren, wobei das Segmentierungsnetzwerk unter Verwendung einer Spezies-Matrix für jedes Material als Ground-Truth trainiert wird, wobei die Spezies-Matrix unter Verwendung der 3D-Elementmatrix für jedes Material bestimmt wird;

Trainieren (312) eines Zellen-Autoencoders unter Verwendung des 3D-Zellenbildes jedes Materials und Erhalten eines Satzes von rekonstruierten Zellenbildern;

Trainieren (314), unter Verwendung des Satzes von rekonstruierten Zellenbildern und des Satzes von rekonstruierten Basisbildern, eines generativen Modells, um einen kontinuierlichen latenten Raum zu erhalten;

Abtasten (316) des kontinuierlichen latenten Raums des generativen Modells, um einen Satz von Zellencodierung und einen Satz von Basiscodierung für ein oder mehrere neue Materialien zu erhalten, wobei das Abtasten unter Verwendung eines von einem zufälligen Abtasten und Interpolieren zwischen latenten Vektoren eines oder mehrerer Materialien aus der Mehrzahl von bekannten Materialien unter Verwendung einer von einer sphärischen und linearen Interpolations-SLERP-Technik durchgeführt wird;

Leiten (318) des Satzes von Zellencodierung durch den Zellen-Autoencoder, um einen Satz von abgetasteten Zellenbildern zu erhalten, und des Satzes von Basiscodierung durch den Basis-Autoencoder, um einen Satz von abgetasteten Basisbildern zu erhalten;

Invertieren (320) des Satzes von abgetasteten Zellenbildern, um einen Satz von Gittervektoren für das eine oder die mehreren neuen Materialien zu erhalten;

Leiten (322) des Satzes von abgetasteten Basisbildern durch das Segmentierungsnetzwerk, um einen Satz von atomaren Clustern zu erhalten, wobei der Satz von atomaren Clustern atomare Positionen und Elementtypen an den atomaren Positionen angibt, und wobei Atomkoordinaten aus dem Satz von atomaren Clustern kombiniert mit dem Satz von Gittervektoren eine Kristallstruktur des einen oder der mehreren neuen Materialien bilden;

Trainieren eines Regressionsmodells zur Vorhersage von Zieleigenschaften des einen oder der mehreren neuen Materialien, wobei der kontinuierliche latente Raum des generativen Modells Merkmale für das Regressionsmodell umfasst; und

Vorhersagen der Zieleigenschaften des einen oder der mehreren neuen Materialien basierend auf der Kristallstruktur des einen oder der mehreren neuen Materialien.

2. Prozessorimplementiertes Verfahren nach Anspruch 1, wobei der Trainingsdatensatz erste Prinzipien von berechneten Strukturen und Eigenschaften der Mehrzahl von bekannten Materialien umfasst.

3. Prozessorimplementiertes Verfahren nach Anspruch 2, wobei der Trainingsdatensatz vorverarbeitet ist, und wobei das Vorverarbeiten des Trainingsdatensatzes umfasst:

Entfernen redundanter Einträge mit derselben Struktur aus dem Trainingsdatensatz; und

Erweitern des Datensatzes durch Erzeugen von Superzellen und Anwenden von zufälligen Translationen und Rotationen auf die Struktur der Mehrzahl von bekannten Materialien.

4. Prozessorimplementiertes Verfahren nach Anspruch 1, wobei das Erhalten der Zellen und Basiscodierungen des einen oder der mehreren neuen Materialien umfasst:

Erhalten eines oder mehrerer latenter Vektoren basierend auf dem Abtasten; und

Erhalten der Zellen und Basiscodierungen des einen oder der mehreren neuen Materialien durch Leiten des einen oder der mehreren latenten Vektoren durch das generative Modell.

5. System (500), umfassend:

einen Speicher (515), der Anweisungen speichert;

eine oder mehrere Kommunikationsschnittstellen (503); und

einen oder mehrere Hardware-Prozessoren (502), die mit dem Speicher (515) über die eine oder die mehreren Kommunikationsschnittstellen (503) gekoppelt sind, wobei der eine oder die mehreren Hardware-Prozessoren (502) durch die Anweisungen konfiguriert sind zum:

Erhalten einer Kristallstruktur jedes Materials einer Mehrzahl von bekannten Materialien aus einem Trainingsdatensatz;

Umwandeln der Kristallstruktur jedes Materials der Mehrzahl von bekannten Materialien in ein dreidimensionales 3D-Zellenbild und ein 3D-Basisbild unter Verwendung einer Mehrzahl von Gaußschen Funktionen;

Erstellen, für jedes 3D-Basisbild jedes Materials, einer 3D-Elementmatrix, die den Ort eines oder mehrerer Elemente in dem 3D-Basisbild jedes Materials darstellt;

Trainieren eines Basis-Autoencoders unter Verwendung des 3D-Basisbildes jedes Materials und Erhalten eines Satzes von rekonstruierten Basisbildern;

Trainieren eines Segmentierungsnetzwerks unter Verwendung des Satzes von rekonstruierten Basisbildern, um einen Ort und Typen eines Satzes von Elementen an diesem Ort als atomare Cluster zu identifizieren, wobei das Segmentierungsnetzwerk unter Verwendung einer Spezies-Matrix für jedes Material als Ground-Truth trainiert wird, wobei die Spezies-Matrix unter Verwendung der 3D-Elementmatrix für jedes Material bestimmt wird;

Trainieren eines Zellen-Autoencoders unter Verwendung des 3D-Zellenbildes jedes Materials und Erhalten eines Satzes von rekonstruierten Zellenbildern;

Trainieren, unter Verwendung des Satzes von rekonstruierten Zellenbildern und des Satzes von rekonstruierten Basisbildern, eines generativen Modells, um einen kontinuierlichen latenten Raum zu erhalten;

Abtasten des kontinuierlichen latenten Raums des generativen Modells, um einen Satz von Zellencodierung und einen Satz von Basiscodierung für ein oder mehrere neue Materialien zu erhalten, wobei das Abtasten unter Verwendung eines von einem zufälligen Abtasten und Interpolieren zwischen latenten Vektoren eines oder mehrerer Materialien aus der Mehrzahl von bekannten Materialien unter Verwendung einer von einer sphärischen und linearen Interpolations-SLERP-Technik durchgeführt wird;

Leiten des Satzes von Zellencodierung durch den Zellen-Autoencoder, um einen Satz von abgetasteten Zellenbildern zu erhalten, und des Satzes von Basiscodierung durch den Basis-Autoencoder, um einen Satz von abgetasteten Basisbildern zu erhalten;

Invertieren des Satzes von abgetasteten Zellenbildern, um einen Satz von Gittervektoren für das eine oder die mehreren neuen Materialien zu erhalten;

Leiten des Satzes von abgetasteten Basisbildern durch das Segmentierungsnetzwerk, um einen Satz von atomaren Clustern zu erhalten, wobei der Satz von atomaren Clustern atomare Positionen und Elementtypen an den atomare Positionen angibt, und wobei Atomkoordinaten aus dem Satz von atomaren Clustern kombiniert mit dem Satz von Gittervektoren eine Kristallstruktur des einen oder der mehreren neuen Materialien bilden;

Trainieren eines Regressionsmodells zur Vorhersage von Zieleigenschaften des einen oder der mehreren neuen Materialien, wobei der kontinuierliche latente Raum des generativen Modells die Merkmale für das Regressionsmodell umfasst; und

Vorhersagen der Zieleigenschaften des einen oder der mehreren neuen Materialien basierend auf der Kristallstruktur des einen oder der mehreren neuen Materialien.

6. System nach Anspruch 5, wobei der Trainingsdatensatz erste Prinzipien von berechneten Strukturen und Eigenschaften der Mehrzahl von bekannten Materialien umfasst.

7. System nach Anspruch 6, wobei der Trainingsdatensatz vorverarbeitet ist, und wobei zum Vorverarbeiten des Trainingsdatensatzes der eine oder die mehreren Hardware-Prozessoren durch die Anweisungen konfiguriert sind zum:

Entfernen redundanter Einträge mit derselben Struktur aus dem Trainingsdatensatz; und

Erweitern des Datensatzes durch Erzeugen von Superzellen und Anwenden von zufälligen Translationen und Rotationen auf die Struktur der Mehrzahl von bekannten Materialien.

8. System nach Anspruch 5, wobei zum Erhalten der Zellen und Basiscodierungen des einen oder der mehreren neuen Materialien der eine oder die mehreren Hardware-Prozessoren durch die Anweisungen konfiguriert sind zum:

Erhalten eines oder mehrerer latenter Vektoren basierend auf dem Abtasten; und

Erhalten der Zellen und Basiscodierungen des einen oder der mehreren neuen Materialien durch Leiten des einen oder der mehreren latenten Vektoren durch das generative Modell.

9. Ein oder mehrere nichtflüchtige maschinenlesbare Informationsspeichermedien, umfassend eine oder mehrere Anweisungen, die, wenn sie von einem oder mehreren Hardware-Prozessoren ausgeführt werden, durch den einen oder die mehreren Hardware-Prozessoren (502) bewirken:

Erhalten einer Kristallstruktur jedes Materials einer Mehrzahl von bekannten Materialien aus einem Trainingsdatensatz;

Umwandeln der Kristallstruktur jedes Materials der Mehrzahl von bekannten Materialien in ein dreidimensionales Zellenbild und ein 3D-Basisbild unter Verwendung einer Mehrzahl von Gaußschen Funktionen;

Erstellen, für jedes 3D-Basisbild jedes Materials, einer 3D-Elementmatrix, die den Ort eines oder mehrerer Elemente in dem 3D-Basisbild jedes Materials darstellt;

Trainieren eines Basis-Autoencoders unter Verwendung des 3D-Basisbildes jedes Materials und Erhalten eines Satzes von rekonstruierten Basisbildern;

Trainieren eines Segmentierungsnetzwerks unter Verwendung des Satzes von rekonstruierten Basisbildern, um einen Ort und Typen eines Satzes von Elementen an diesem Ort als atomare Cluster zu identifizieren, wobei das Segmentierungsnetzwerk unter Verwendung einer Spezies-Matrix für jedes Material als Ground-Truth trainiert wird, wobei die Spezies-Matrix unter Verwendung der 3D-Elementmatrix für jedes Material bestimmt wird;

Trainieren eines Zellen-Autoencoders unter Verwendung des 3D-Zellenbildes jedes Materials und Erhalten eines Satzes von rekonstruierten Zellenbildern;

Trainieren, unter Verwendung des Satzes von rekonstruierten Zellenbildern und des Satzes von rekonstruierten Basisbildern, eines generativen Modells, um einen kontinuierlichen latenten Raum zu erhalten;

Abtasten des kontinuierlichen latenten Raums des generativen Modells, um einen Satz von Zellencodierung und einen Satz von Basiscodierung für ein oder mehrere neue Materialien zu erhalten, wobei das Abtasten unter Verwendung eines von einem zufälligen Abtasten und Interpolieren zwischen latenten Vektoren eines oder mehrerer Materialien aus der Mehrzahl von bekannten Materialien unter Verwendung einer von einer sphärischen und linearen Interpolations-SLERP-Technik durchgeführt wird;

Leiten des Satzes von Zellencodierung durch den Zellen-Autoencoder, um einen Satz von abgetasteten Zellenbildern zu erhalten, und des Satzes von Basiscodierung durch den Basis-Autoencoder, um einen Satz von abgetasteten Basisbildern zu erhalten;

Invertieren des Satzes von abgetasteten Zellenbildern, um einen Satz von Gittervektoren für das eine oder die mehreren neuen Materialien zu erhalten; und

Leiten des Satzes von abgetasteten Basisbildern durch das Segmentierungsnetzwerk, um einen Satz von atomaren Clustern zu erhalten, wobei der Satz von atomaren Clustern atomare Positionen und Elementtypen an den atomare Positionen angibt, und wobei Atomkoordinaten aus dem Satz von atomaren Clustern kombiniert mit dem Satz von Gittervektoren eine Kristallstruktur des einen oder der mehreren neuen Materialien bilden;

Trainieren eines Regressionsmodells zur Vorhersage von Zieleigenschaften des einen oder der mehreren neuen Materialien, wobei der kontinuierliche latente Raum des generativen Modells Merkmale für das Regressionsmodell umfasst; und

Vorhersagen der Zieleigenschaften des einen oder der mehreren neuen Materialien basierend auf der Kristallstruktur des einen oder der mehreren neuen Materialien.

## Revendications

1. Procédé (300) mis en oeuvre par processeur matériel (502), comprenant :

l'obtention (302) d'une structure cristalline de chaque matériau d'une pluralité de matériaux connus à partir d'un ensemble de données d'entraînement ;

la conversion (304) de la structure cristalline de chaque matériau de la pluralité de matériaux connus en une image de cellule tridimensionnelle (3D) et une image de base 3D en utilisant une pluralité de fonctions gaussiennes ;

la création (306), pour chaque image de base 3D de chaque matériau, d'une matrice d'éléments 3D représentant l'emplacement d'un ou plusieurs éléments dans l'image de base 3D de chaque matériau ;

l'entraînement (308) d'un auto-codeur de base en utilisant l'image de base 3D de chaque matériau et l'obtention d'un ensemble d'images de base reconstruites ;

l'entraînement (310) d'un réseau de segmentation en utilisant l'ensemble d'images de base reconstruites pour identifier un emplacement et des types d'un ensemble d'éléments à cet emplacement en tant que clusters atomiques, dans lequel le réseau de segmentation est entraîné en utilisant une matrice d'espèces pour chaque matériau en tant que réalité de terrain, dans lequel la matrice d'espèces est déterminée en utilisant la matrice d'éléments 3D pour chaque matériau ;

l'entraînement (312) d'un auto-codeur de cellule en utilisant l'image de cellule 3D de chaque matériau et l'obtention d'un ensemble d'images de cellule reconstruites ;

l'entraînement (314), en utilisant l'ensemble d'images de cellule reconstruites et l'ensemble d'images de base reconstruites, d'un modèle génératif pour obtenir un espace latent continu ;

l'échantillonnage (316) de l'espace latent continu du modèle génératif pour obtenir un ensemble d'encodage de cellules et un ensemble d'encodage de base pour un ou plusieurs nouveaux matériaux, dans lequel l'échantillonnage est effectué en utilisant un échantillonnage aléatoire et une interpolation entre des vecteurs latents d'un ou plusieurs matériaux parmi la pluralité de matériaux connus en utilisant l'une des techniques d'interpolation sphérique et linéaire-SLERP ;

le passage (318) de l'ensemble d'encodage de cellules à travers l'auto-codeur de cellule pour obtenir un ensemble d'images de cellule échantillonnées et de l'ensemble d'encodage de base à travers l'auto-codeur de base pour obtenir un ensemble d'images de base échantillonnées ;

l'inversion (320) de l'ensemble d'images de cellule échantillonnées pour obtenir un ensemble de vecteurs de réseau pour un ou plusieurs nouveaux matériaux ;

le passage (322) de l'ensemble d'images de base échantillonnées à travers le réseau de segmentation pour obtenir un ensemble de clusters atomiques, dans lequel l'ensemble de clusters atomiques indique des positions atomiques et des types d'éléments aux positions atomiques, et dans lequel des coordonnées atomiques de l'ensemble de clusters atomiques combinées avec l'ensemble de vecteurs de réseau constituent une structure cristalline d'un ou plusieurs nouveaux matériaux ;

l'entraînement d'un modèle de régression pour une prédiction de propriétés cibles d'un ou plusieurs nouveaux matériaux, dans lequel l'espace latent continu du modèle génératif comprend des caractéristiques pour le modèle de régression ; et

la prédiction des propriétés cibles d'un ou plusieurs nouveaux matériaux sur la base de la structure cristalline d'un ou plusieurs nouveaux matériaux.

2. Procédé mis en oeuvre par processeur selon la revendication 1, dans lequel l'ensemble de données d'entraînement comprend des structures et des propriétés calculées à partir de premiers principes pour la pluralité de matériaux connus.

3. Procédé mis en oeuvre par processeur selon la revendication 2, dans lequel l'ensemble de données d'entraînement est prétraité, et dans lequel le prétraitement de l'ensemble de données d'entraînement comprend :

la suppression d'entrées redondantes ayant la même structure de l'ensemble de données d'entraînement ; et l'augmentation de l'ensemble de données en créant des supercellules et en appliquant des translations et des rotations aléatoires à la structure de la pluralité de matériaux connus.

4. Procédé mis en oeuvre par processeur selon la revendication 1, dans lequel l'obtention des codages cellulaires et de base d'un ou plusieurs nouveaux matériaux comprend :

l'obtention d'un ou plusieurs vecteurs latents sur la base de l'échantillonnage ; et l'obtention des codages cellulaires et de base d'un ou plusieurs nouveaux matériaux en faisant passer un ou plusieurs vecteurs latents à travers le modèle génératif.

5. Système (500), comprenant :

une mémoire (515) stockant des instructions ;
une ou plusieurs interfaces de communication (503) ; et
un ou plusieurs processeurs matériels (502) couplés à la mémoire (515) via une ou plusieurs interfaces de communication (503), dans lequel un ou plusieurs processeurs matériels (502) sont configurés par les instructions pour :

l'obtention d'une structure cristalline de chaque matériau d'une pluralité de matériaux connus à partir d'un ensemble de données d'entraînement ;
la conversion de la structure cristalline de chaque matériau de la pluralité de matériaux connus en une image de cellule tridimensionnelle (3D) et une image de base 3D en utilisant une pluralité de fonctions gaussiennes ;
la création, pour chaque image de base 3D de chaque matériau, d'une matrice d'éléments 3D représentant l'emplacement d'un ou plusieurs éléments dans l'image de base 3D de chaque matériau ;
l'entraînement d'un auto-codeur de base en utilisant l'image de base 3D de chaque matériau et l'obtention d'un ensemble d'images de base reconstruites ;
l'entraînement d'un réseau de segmentation en utilisant l'ensemble d'images de base reconstruites pour identifier un emplacement et des types d'un ensemble d'éléments à cet emplacement en tant que clusters atomiques, dans lequel le réseau de segmentation est entraîné en utilisant une matrice d'espèces pour chaque matériau en tant que réalité de terrain, dans lequel la matrice d'espèces est déterminée en utilisant la matrice d'éléments 3D pour chaque matériau ;
l'entraînement d'un auto-codeur de cellule en utilisant l'image de cellule 3D de chaque matériau et l'obtention d'un ensemble d'images de cellule reconstruites ;
l'entraînement, en utilisant l'ensemble d'images de cellule reconstruites et l'ensemble d'images de base reconstruites, d'un modèle génératif pour obtenir un espace latent continu ;
l'échantillonnage de l'espace latent continu du modèle génératif pour obtenir un ensemble d'encodage de cellules et un ensemble d'encodage de base pour un ou plusieurs nouveaux matériaux, dans lequel l'échantillonnage est effectué en utilisant un échantillonnage aléatoire et une interpolation entre des vecteurs latents d'un ou plusieurs matériaux parmi la pluralité de matériaux connus en utilisant l'une des techniques d'interpolation sphérique et linéaire-SLERP ;
le passage de l'ensemble d'encodage de cellules à travers l'auto-codeur de cellule pour obtenir un ensemble d'images de cellule échantillonnées et de l'ensemble d'encodage de base à travers l'auto-codeur de base pour obtenir un ensemble d'images de base échantillonnées ;

l'inversion de l'ensemble d'images de cellule échantillonnées pour obtenir un ensemble de vecteurs de réseau pour un ou plusieurs nouveaux matériaux ;

le passage de l'ensemble d'images de base échantillonnées à travers le réseau de segmentation pour obtenir un ensemble de clusters atomiques, dans lequel l'ensemble de clusters atomiques indique des positions atomiques et des types d'éléments aux positions atomiques, et dans lequel des coordonnées atomiques de l'ensemble de clusters atomiques combinées avec l'ensemble de vecteurs de réseau constituent une structure cristalline d'un ou plusieurs nouveaux matériaux ;

l'entraînement d'un modèle de régression pour une prédiction de propriétés cibles d'un ou plusieurs nouveaux matériaux, dans lequel l'espace latent continu du modèle génératif comprend des caractéristiques pour le modèle de régression ; et

la prédiction des propriétés cibles d'un ou plusieurs nouveaux matériaux sur la base de la structure cristalline d'un ou plusieurs nouveaux matériaux.

6. Système selon la revendication 5, dans lequel l'ensemble de données d'entraînement comprend des structures et des propriétés calculées à partir de premiers principes pour la pluralité de matériaux connus.

7. Système selon la revendication 6, dans lequel l'ensemble de données d'entraînement est prétraité, et dans lequel pour prétraiter l'ensemble de données d'entraînement, un ou plusieurs processeurs matériels sont configurés par les instructions pour :

la suppression d'entrées redondantes ayant la même structure de l'ensemble de données d'entraînement ; et l'augmentation de l'ensemble de données en créant des supercellules et en appliquant des translations et des rotations aléatoires à la structure de la pluralité de matériaux connus.

8. Système selon la revendication 5, dans lequel pour obtenir des codages cellulaires et de base d'un ou plusieurs nouveaux matériaux, un ou plusieurs processeurs matériels sont configurés par les instructions pour :

l'obtention d'un ou plusieurs vecteurs latents sur la base de l'échantillonnage ; et l'obtention des codages cellulaires et de base d'un ou plusieurs nouveaux matériaux en faisant passer un ou plusieurs vecteurs latents à travers le modèle génératif.

9. Un ou plusieurs supports de stockage d'informations lisibles par machine non transitoires comprenant une ou plusieurs instructions qui, lorsqu'elles sont exécutées par un ou plusieurs processeurs matériels, amènent un ou plusieurs processeurs matériels (502) à :

l'obtention d'une structure cristalline de chaque matériau d'une pluralité de matériaux connus à partir d'un ensemble de données d'entraînement ;

la conversion de la structure cristalline de chaque matériau de la pluralité de matériaux connus en une image de cellule tridimensionnelle (3D) et une image de base 3D en utilisant une pluralité de fonctions gaussiennes ;

la création, pour chaque image de base 3D de chaque matériau, d'une matrice d'éléments 3D représentant l'emplacement d'un ou plusieurs éléments dans l'image de base 3D de chaque matériau ;

l'entraînement d'un auto-codeur de base en utilisant l'image de base 3D de chaque matériau et l'obtention d'un ensemble d'images de base reconstruites ;

l'entraînement d'un réseau de segmentation en utilisant l'ensemble d'images de base reconstruites pour identifier un emplacement et des types d'un ensemble d'éléments à cet emplacement en tant que clusters atomiques, dans lequel le réseau de segmentation est entraîné en utilisant une matrice d'espèces pour chaque matériau en tant que réalité de terrain, dans lequel la matrice d'espèces est déterminée en utilisant la matrice d'éléments 3D pour chaque matériau ;

l'entraînement d'un auto-codeur de cellule en utilisant l'image de cellule 3D de chaque matériau et l'obtention d'un ensemble d'images de cellule reconstruites ;

l'entraînement, en utilisant l'ensemble d'images de cellule reconstruites et l'ensemble d'images de base reconstruites, d'un modèle génératif pour obtenir un espace latent continu ;

l'échantillonnage de l'espace latent continu du modèle génératif pour obtenir un ensemble d'encodage de cellules et un ensemble d'encodage de base pour un ou plusieurs nouveaux matériaux, dans lequel l'échantillonnage est effectué en utilisant un échantillonnage aléatoire et une interpolation entre des vecteurs latents d'un ou plusieurs matériaux parmi la pluralité de matériaux connus en utilisant l'une des techniques d'interpolation sphérique et linéaire-SLERP ;

le passage de l'ensemble d'encodage de cellules à travers l'auto-codeur de cellule pour obtenir un ensemble d'images de cellule échantillonnées et de l'ensemble d'encodage de base à travers l'auto-codeur de base pour obtenir un ensemble d'images de base échantillonnées ;

l'inversion de l'ensemble d'images de cellule échantillonnées pour obtenir un ensemble de vecteurs de réseau pour un ou plusieurs nouveaux matériaux ;

le passage de l'ensemble d'images de base échantillonnées à travers le réseau de segmentation pour obtenir un ensemble de clusters atomiques, dans lequel l'ensemble de clusters atomiques indique des positions atomiques et des types d'éléments aux positions atomiques, et dans lequel des coordonnées atomiques de l'ensemble de clusters atomiques combinées avec l'ensemble de vecteurs de réseau constituent une structure cristalline d'un ou plusieurs nouveaux matériaux ;

l'entraînement d'un modèle de régression pour une prédiction de propriétés cibles d'un ou plusieurs nouveaux matériaux, dans lequel l'espace latent continu du modèle génératif comprend des caractéristiques pour le modèle de régression ; et

la prédiction des propriétés cibles d'un ou plusieurs nouveaux matériaux sur la base de la structure cristalline d'un ou plusieurs nouveaux matériaux.

FIG. 1

FIG. 2

OBTAIN CRYSTAL STRUCTURE OF EACH OF A PLURALITY OF MATERIALS OBTAINED IN A TRAINING DATA SET, VIA ONE OR MORE HARDWARE PROCESSORS ⌐ 302

CONVERT THE CRYSTAL STRUCTURE OF EACH MATERIAL OF THE PLURALITY OF MATERIALS INTO A THREE-DIMENSIONAL (3D) CELL IMAGE AND A 3D BASIS IMAGE USING A PLURALITY OF GAUSSIAN FUNCTIONS, VIA THE ONE OR MORE HARDWARE PROCESSORS ⌐ 304

CREATE, FOR EACH 3D BASIS IMAGE OF THE EACH MATERIAL, A 3D ELEMENTS MATRIX REPRESENTING LOCATION OF ONE OR MORE ELEMENTS IN THE 3D BASIS IMAGE OF THE MATERIAL, VIA THE ONE OR MORE HARDWARE PROCESSORS ⌐ 306

TRAIN A BASIS AUTOENCODER USING THE 3D BASIS IMAGE OF THE EACH MATERIAL AND OBTAINING A SET OF RECONSTRUCTED BASIS IMAGES, VIA THE ONE OR MORE HARDWARE PROCESSORS ⌐ 308

TRAIN, VIA THE ONE OR MORE HARDWARE PROCESSORS, A SEGMENTATION NETWORK USING THE SET OF RECONSTRUCTED BASIS IMAGES TO IDENTIFY LOCATION AND TYPES OF A SET OF ELEMENTS AT THE LOCATIONS AS ATOMIC CLUSTERS, THE SEGMENTATION NETWORK TRAINED BY USING A SPECIES MATRIX FOR EACH MATERIAL AS A GROUND TRUTH; WHEREIN THE SPECIES MATRIX IS DETERMINED USING THE 3D ELEMENTS MATRIX FOR THE MATERIAL ⌐ 310

TRAIN A CELL AUTOENCODER USING THE 3D CELL IMAGE OF THE EACH MATERIAL AND OBTAINING A SET OF RECONSTRUCTED CELL IMAGES, VIA THE ONE OR MORE HARDWARE PROCESSORS ⌐ 312

A

← 300

FIG. 3A

```
                              ( A )
                                │
                                ▼
```

TRAIN, USING THE SET OF RECONSTRUCTED CELL IMAGES AND THE SET OF RECONSTRUCTED BASIS IMAGES, A GENERATIVE MODEL TO OBTAIN A CONTINUOUS LATENT SPACE, VIA THE ONE OR MORE HARDWARE PROCESSORS ⟋314

SAMPLE, VIA THE ONE OR MORE HARDWARE PROCESSORS, THE CONTINUOUS LATENT SPACE OF THE GENERATIVE MODEL TO OBTAIN A SET OF CELL ENCODING AND A SET OF BASIS ENCODING FOR ONE OR MORE NEW MATERIALS ASSOCIATED WITH ONE OR MORE CONDITIONS OF THE APPLICATION, THE SAMPLING PERFORMED USING ONE OF A RANDOM SAMPLING OR INTERPOLATING BETWEEN LATENT VECTORS OF ONE OR MORE MATERIALS FROM AMONGST THE PLURALITY OF KNOWN MATERIALS USING ONE OF A SPHERICAL AND LINEAR INTERPOLATION (SLERP) TECHNIQUES ⟋316

PASS. VIA THE ONE OR MORE HARDWARE PROCESSORS, THE SET OF CELL ENCODING THROUGH THE CELL AUTOENCODER TO OBTAIN A SET OF CELL IMAGES AND THE SET OF BASIS ENCODINGS THROUGH THE BASIS AUTOENCODER TO OBTAIN A SET OF BASIS IMAGES ⟋318

INVERT THE SET OF CELL IMAGES TO OBTAIN A SET OF LATTICE VECTORS FOR THE NEW MATERIAL, VIA THE ONE OR MORE HARDWARE PROCESSORS ⟋320

PASSING, VIA THE ONE OR MORE HARDWARE PROCESSORS, THE SET OF BASIS IMAGES THROUGH THE SEGMENTATION NETWORK TO OBTAIN A SET OF ATOMIC CLUSTERS, WHEREIN THE SET OF ATOMIC CLUSTERS ARE INDICATIVE OF ATOMIC POSITIONS AND ELEMENT TYPES AT THE ATOMIC POSITIONS, AND WHEREIN ATOM COORDINATES FROM THE SET OF ATOMIC CLUSTERS COMBINED WITH THE SET OF LATTICE VECTORS CONSTITUTES THE CRYSTAL STRUCTURE OF THE NEW MATERIAL ⟋322

## FIG. 3B ⟵ 300

**STEP 1** — **CONVERSION OF CRYSTAL STRUCTUTES INTO 3D IMAGES**

CRYSTAL STRUCTURE → CELL — GAUSSIAN FUNCTION → 3D CELL IMAGE

CRYSTAL STRUCTURE → BASIS — ATOMIC NUMBER WEIGHTED GAUSSIAN FUNCTION → 3D BASIS IIMAGE → ELEMENTS MATRIX

**STEP 2** — **TRAINING OF AUTOENCODERS, SEGEMENTATION NETWORK**

3D CELL IMAGE → CELL ENCODER → CELL ENCODINGS → CELL DECODER → RECONSTRUCTED CELL IMAGE

3D BASIS IMAGE → BASIS ENCODER → BASIS ENCODINGS → BASIS DECODER → RECONSTRUCTED BASIS IMAGE

RECONSTRUCTED BASIS IMAGE → 3D SEGEMENTATION NETWORK → SPECIES MATRIX

**STEP 3** — **GENERATIVE MODEL TRAINING**

CELL ENCODINGS, BASIS ENCODINGS → GENERATIVE MODEL → LATENT SPACE REPRESENATION → DECODER → RECONSTRUCTED CELL ENCODINGS / RECONSTRUCTED BASIS ENCODINGS

FIG. 4A     ← 400

EP 4 071 657 B1

NEW MATERIAL GENERATION

STEP 4

CELL ENCODINGS → CELL DECODER

INVERSE TRANSFORMATION ← CELL IMAGE

MEAN ENCODINGS → SLERP/RANDOM SAMPLING → NEW LATENT VECTOR → DECODER

NEW MATERIAL

ATOMS, POSITIONS

COORDINATE CONVERTOR

BASIS ENCODINGS → BASIS DECODER → BASIS IMAGE → 3D SEGMENTATION NETWORK → SPECIES MATRIX

STEP 5    NEW MATERIAL PROPERTY PREDICTION

NEW MATERIAL → REGRESSION MODEL → PROPERTY VALUE

FIG. 4B

400

EP 4 071 657 B1

FIG. 5

CRYSTAL
STRUCTURE

CELL IMAGE
2D SLICE

BASIS IMAGE
2D SLICE

ELEMENTS MATRIX

FIG. 6

EP 4 071 657 B1

INPUT CELL IMAGE    OUTPUT CELL IMAGE

FIG. 7

INPUT BASIS IMAGE    OUTPUT BASIS IMAGE

INPUT ELEMENTS MATRIX    OUTPUT ELEMENTS MATRIX

FIG. 8

FIG. 9

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202121016622

**Non-patent literature cited in the description**

- **MATTER.** *Inverse Design of Solid-State Materials via a Continuous Representation,* 06 November 2019, vol. 1 (5), ISSN 2590-2385, 1370-1384
- CCDCGAN: Inverse design of crystal structures. ARXIV.ORG. CORNELL UNIVERSITY LIBRARY
- Generative Adversarial Networks for Crystal Structure Prediction. ARXIV.ORG. CORNELL UNIVERSITY LIBRARY
- Machine learning for crystal identification and discovery. AICHE JOURNAL. JOHN WILEY & SONS, INC
- High-throughput discovery of novel cubic crystal materials using deep generative neural networks. ARXIV.ORG. CORNELL UNIVERSITY LIBRARY
- Inverse design of crystals using generalized invertible crystallographic representation. ARXIV.ORG. CORNELL UNIVERSITY LIBRARY
- CrystalGAN: Learning to Discover Crystallographic Structures with Generative Adversarial Networks. ARXIV.ORG. CORNELL UNIVERSITY LIBRARY